# EUROPÄISCHE PATENTSCHRIFT

(11) **EP 4 444 454 B1**
(45) Veröffentlichungstag und Bekanntmachung des Hinweises auf die Patenterteilung: **17.06.2026**
(21) Anmeldenummer: 22830816.9
(22) Anmeldetag: 07.12.2022
(51) Int. Cl.: B01F 27/054, B01F 27/07, C12M 1/06, C12M 1/34

(54) **RÜHRWERK FÜR EINEN BIOREAKTOR, RÜHREINHEIT, BIOREAKTOR, HERSTELLUNGSVERFAHREN UND VERFAHREN ZUM BETREIBEN EINES BIOREAKTORS**
AGITATOR FOR A BIOREACTOR, STIRRING UNIT, BIOREACTOR, MANUFACTURING METHOD AND METHOD FOR OPERATING A BIOREACTOR
MÉCANISME D'AGITATION POUR UN BIORÉACTEUR, UNITÉ D'AGITATION, BIORÉACTEUR, PROCÉDÉ DE FABRICATION ET PROCÉDÉ DE FONCTIONNEMENT D'UN BIORÉACTEUR

(30) Priorität: 07.12.2021 EP 21212832
(43) Veröffentlichungstag der Anmeldung: 16.10.2024
(73) Patentinhaber: Eppendorf SE, 22339 Hamburg (DE)
(72) Erfinder: SCHÜTT, Anna, 22299 Hamburg (DE)
(74) Vertreter: RGTH
(86) Internationale Anmeldenummer: PCT/EP2022/084739
(87) Internationale Veröffentlichungsnummer: WO 2023/104859

(56) Entgegenhaltungen:
- EP-A1- 0 865 817
- WO-A1-2020/221790
- DE-A1- 102006 053 339
- DE-A1- 19 859 511
- FR-A1- 2 685 220
- US-B2- 8 753 005

## Beschreibung

Die Erfindung betrifft ein Rührwerk für einen Bioreaktor, eine Rühreinheit zum Aufrüsten einer Rührwelle sowie einen Bioreaktor. Die Erfindung betrifft zudem ein Verfahren zum Herstellen einer Rühreinheit und/oder eines Rührwerks für einen Bioreaktor, ein Verfahren zum Herstellen eines Bioreaktors sowie ein Verfahren zum Betreiben eines Bioreaktors.

Bioreaktoren, häufig auch als Fermenter bezeichnet, sind Behältersysteme, in denen bestimmte Mikroorganismen, Zellen oder Pflanzen, vorzugsweise unter möglichst optimalen, kontrollierten und reproduzierbaren Bedingungen, kultiviert werden, um Zellen selbst oder Teile von ihnen oder eines ihrer Stoffwechselprodukte zu gewinnen. Hierzu umschließen Bioreaktoren einen Reaktionsraum, in dem ein Medium, insbesondere ein Kulturmedium, aufgenommen werden kann.

Es sind verschiedene Arten von Bioreaktoren, wie beispielsweise die sogenannten Rührkesselreaktoren, auch als Rührreaktor bezeichnet, bekannt. Derartige Bioreaktoren weisen unterschiedlich ausgeführte Rührfunktionen zur Vermischung und insbesondere zur Homogenisierung der im Reaktionsraum vorhandenen Stoffe, insbesondere des Kulturmediums, auf. Die Vermischung und insbesondere die Homogenisierung erfolgt hierbei beispielsweise über eine Drehbewegung mindestens eines an einer Rührwelle angebrachten Rührblatts. Die Rührwelle wird hierzu insbesondere von einer Antriebseinheit in Rotation versetzt.

Unterschiedliche Rührertypen und deren Eigenschaften sind bekannt. Die Auswahl eines geeigneten Rührertyps richtet sich insbesondere nach einer Rühraufgabe, einer Zähigkeit des Kulturmediums, einer Scherfestigkeit oder auch danach, welche Rührerleistung zur Verfügung steht bzw. für den Prozess erforderlich ist. Beispielsweise können folgende wesentliche Rühraufgaben unterschieden werden: Homogenisieren, Dispergieren, Wärmeübergang, Suspendieren und Emulgieren. Diese Rühraufgaben treten selten isoliert auf, sodass die Notwendigkeit besteht, verschiedene Rühraufgaben gleichzeitig oder in einer zeitlichen Folge nacheinander umzusetzen.

Für die unterschiedlichen Rühraufgaben sind in der Regel unterschiedliche Rührertypen geeignet bzw. bevorzugt. Daher muss bei der Auswahl eines Rührertyps häufig ein Kompromiss geschlossen werden zwischen einem "optimalen" Rührertyp für wesentliche Prozessschritte und einer generellen Eignung für ebenfalls auftretende, aber für die Ausbeute und die Qualität des Produkts weniger relevanten Verfahrensschritte.

US 8 753 005 B2 beschreibt eine Mischapparatur mit Rührer. US 8 753 005 B2 offenbart dabei insbesondere in den Abbildungen 13A-13C eine Rühreinheit für eine Rührwelle, umfassend einen Rührblattaufnahmeabschnitt und ein Rührblatt, das an dem Rührblattaufnahmeabschnitt angeordnet und zwischen einer ersten Winkelposition und einer zweiten Winkelposition bewegbar ist, wobei das Rührblatt in der ersten Winkelposition in einem ersten Anstellwinkel und in der zweiten Winkelposition in einem zweiten Anstellwinkel ausgerichtet ist, wobei das Rührblatt ein drehsteifes Rotationselement umfasst, das zwischen einer ersten Drehposition und einer zweiten Drehposition drehbar an dem Rührblattaufnahmeabschnitt gelagert ist, wobei das Rührblatt in der ersten Drehposition in dem ersten Anstellwinkel und in der zweiten Drehposition in dem zweiten Anstellwinkel ausgerichtet ist, wobei der Rührblattaufnahmeabschnitt mindestens eine Aufnahmeöffnung aufweist, in der das Rotationselement zwischen der ersten Drehposition und der zweiten Drehposition drehbar gelagert aufgenommen ist, weiter umfassend einen ersten Anschlag, der eine Bewegung des mindestens einen Rührblatts in der ersten Winkelposition in eine erste Bewegungsrichtung begrenzt.

In der Regel sind Bioreaktoren in einer kontinuierlichen Prozessführung zu betreiben.

Insbesondere zur Vermeidung eines Kontaminationsrisikos werden Rühreinheiten während des Betriebs im Regelfall nicht ausgetauscht. Herausfordernd für die Auswahl eines Rührertyps ist diesbezüglich auch die Tatsache, dass sich oftmals die Eigenschaften des Kulturmediums während eines Prozesses, beispielsweise von einem niedrigviskosen Newton'schen Verhalten hin zu stark strukturviskosem nicht-Newton'schen Fließverhalten, verändern. Dies kann insbesondere zu einer ungleichmäßigen Verteilung der Viskosität innerhalb des Bioreaktors führen. Dadurch können beispielsweise eine Homogenisierung und ein Massentransport negativ beeinflusst werden.

Zudem werden derartige Rührwerke auch in Einwegbioreaktoren ("Single-Use" Bioreaktor) verbaut. Hierbei wird in der Regel ein einziger Rührertyp ausgewählt und somit ist auch nur ein bestimmter Rührertyp im Prozess nutzbar. Soll demnach ein bestimmtes Medium im Bioreaktor prozessiert werden, muss sich der Nutzer im Vorfeld für einen Rührertyp entscheiden. Der Nutzer kann während des Prozesses die Fluidförderung also nicht mehr variieren und insbesondere an sich ändernde Parameter anpassen.

Der Erfindung liegt daher die Aufgabe zugrunde, eine verbesserte Lösung bereitzustellen, welche wenigstens eines der genannten Probleme adressiert. Insbesondere ist es eine Aufgabe der Erfindung, eine Lösung bereitzustellen, die unterschiedliche Rühreigenschaften bedarfsabhängig erfüllen kann und gleichzeitig eine kontinuierliche Prozessführung ermöglicht.

Gemäß einem ersten Aspekt wird die eingangs genannte Aufgabe gelöst durch ein Rührwerk für einen Bioreaktor gemäß Anspruch 1, umfassend eine Rührwelle mit einer Rühreinheit mit einem Rührblattaufnahmeabschnitt und mindestens einem Rührblatt, das an dem Rührblattaufnahmeabschnitt angeordnet und zwischen einer ersten Winkelposition und einer zweiten Winkelposition bewegbar ist, wobei das mindestens eine Rührblatt in der ersten Winkelposition in einem ersten Anstellwinkel und in der zweiten Winkelposition in einem zweiten Anstellwinkel ausgerichtet ist. Dabei umfasst das mindestens eine Rührblatt ein drehsteifes Rotationselement, das zwischen einer ersten Drehposition und einer zweiten Drehposition drehbar an dem Rührblattaufnahmeabschnitt gelagert ist. Das mindestens eine Rührblatt ist in der ersten Drehposition in dem ersten Anstellwinkel und in der zweiten Drehposition in dem zweiten Anstellwinkel ausgerichtet. Der Rührblattaufnahmeabschnitt weist mindestens eine Aufnahmeöffnung auf, in der das Rotationselement zwischen der ersten Drehposition und der zweiten Drehposition drehbar gelagert aufgenommen ist. Die Rühreinheit umfasst weiter einen ersten Anschlag, der eine Bewegung des mindestens einen Rührblatts in der ersten Winkelposition in eine erste Bewegungsrichtung begrenzt, und/oder einen zweiten Anschlag, der eine Bewegung des mindestens einen Rührblatts in der zweiten Winkelposition in eine zweite Bewegungsrichtung begrenzt, wobei vorzugsweise der erste Anschlag und/oder der zweite Anschlag an dem Rührblattaufnahmeabschnitt angeordnet oder durch diesen ausgebildet ist, wobei die Aufnahmeöffnung den ersten Anschlag und/oder den zweiten Anschlag bildet oder durch den ersten Anschlag und/oder den zweiten Anschlag begrenzt ist.

Der Erfindung liegt unter anderem die Erkenntnis zugrunde, dass in Abhängigkeit zu erzielender Rühreigenschaften unterschiedliche Rührer, wie zum Beispiel Schrägblattrührer und Scheibenrührer, zum Einsatz kommen. Schrägblattrührer umfassen in der Regel schräg angestellte, meist rechtwinklige Rührblätter. Zudem sind Schrägblattrührer im Wesentlichen axial fördernd mit radialen Anteil. Dadurch können Schrägblattrührer insbesondere für ein Suspendieren, ein Homogenisieren und/oder einen Wärmeaustausch eingesetzt werden. Schrägblattrührer werden insbesondere bevorzugt bei schersensitiven Prozessen eingesetzt. Scheibenrührer, auch "Rushton-Turbine" genannt, umfassen in der Regel senkrecht stehende Rührblätter. Die Rührwirkung beruht hierbei insbesondere auf einer auswärts gerichteten Strömung mit einer axialen Ansaugung von oben und unten. Die abströmende Flüssigkeit unterliegt hierbei in der Regel einer hohen Scherung. Die radiale Förderung kann insbesondere zum Emulgieren und Begasen vorteilhaft eingesetzt werden.

In der hier beschriebenen Lösung wird ein Rührwerk mit einer Rührwelle und einer Rühreinheit bereitgestellt, das unterschiedliche Rührertypen in einem Rührwerk verwirklicht. In Abhängigkeit einer gewünschten Rühreigenschaft kann zwischen zwei Rührertypen ausgewählt und insbesondere bedarfsabhängig gewechselt werden. Das Wechseln zwischen zwei Rührertypen wird insbesondere dadurch ermöglicht, dass das mindestens eine Rührblatt an dem Rührblattaufnahmeabschnitt derart angeordnet ist, dass dieses zwischen der ersten Winkelposition und der zweiten Winkelposition hin und her bewegt werden kann, um so das mindestens eine Rührblatt in verschiedenen Anstellwinkeln ausrichten zu können. So können beispielweise ein Schrägblattrührer und ein Scheibenrührer in einem Rührwerk und/oder in einer Rühreinheit integriert sein und in Abhängigkeit des Bedarfs zum Einsatz kommen.

Dadurch, dass das Rührwerk zwei unterschiedliche Rührertypen in einem Rührwerk realisiert, kann der Anwender insbesondere in Abhängigkeit eines Bedarfs sich zwischen diesen zwei Rührertypen während eines kontinuierlichen Prozesses entscheiden. Dem Nutzer wird dadurch ein Wechsel zwischen diesen Rührertypen innerhalb eines kontinuierlichen Prozesses ermöglicht, um dadurch eine Fluidförderung, insbesondere eine Fluidförderrichtung, während dieses Prozesses zu verändern und insbesondere an das zu fördernde Medium anzupassen.

Vorzugsweise kann die Rühreinheit an der Rührwelle angeordnet oder ausgebildet sein. Rühreinheiten können insbesondere je nach Ausführungen und Baugröße samt Rührwelle getauscht werden oder an der Rührwelle in Abhängigkeit der gewünschten Rühreigenschaften montiert werden. Insbesondere kann die Rühreinheit lösbar an der Rührwelle befestigt sein. Dadurch kann die Rühreinheit in Abhängigkeit der gewünschten Rühreigenschaften ausgetauscht werden. Durch diese Ausgestaltung kann beispielsweise auch ein Aufrüsten einer Rührwelle ermöglicht werden.

Der Rührblattaufnahmeabschnitt kann dabei beispielweise ein Teil der Rührwelle sein und/oder durch die Rührwelle selbst ausgebildet sein. Alternativ kann der Rührblattaufnahmeabschnitt als zusätzliches Element an der Rührwelle angeordnet werden.

Unter einem Rührblattaufnahmeabschnitt kann insbesondere ein Abschnitt verstanden werden, an dem das mindestens eine Rührblatt angeordnet ist. Das mindestens eine Rührblatt kann hierbei entweder an dem Rührblattaufnahmeabschnitt befestigt werden oder an diesem ausgebildet sein. Der Rührblattaufnahmeabschnitt kann hierbei insbesondere derart ausgebildet sein, dass das mindestens eine Rührblatt bewegbar an dem Rührblattaufnahmeabschnitt angeordnet, insbesondere gelagert, ist.

Vorzugsweise können zwei, drei oder mehrere Rührblätter an dem Rührblattaufnahmeabschnitt angeordnet sein. Insbesondere bevorzugt können insbesondere sechs Rührblätter an dem Rührblattaufnahmeabschnitt angeordnet sein. Dadurch werden besonders bevorzugte Rühreigenschaften erzielt.

Ein Rührblatt kann hierbei insbesondere rechtwinklig, vorzugsweise rechteckig, ausgebildet sein. Alternativ sind auch andere Formen denkbar. Beispielsweise ist auch eine Form mit abgerundeten Ecken denkbar. Insbesondere erstreckt sich das mindestens eine Rührblatt ausgehend von dem Rührblattaufnahmeabschnitt und somit relativ zu einer Längsachse der Rührwelle nach außen.

Unter einem Anstellwinkel des mindestens einen Rührblatts kann vorliegend insbesondere ein Winkel zwischen dem mindestens einem Rührblatt, insbesondere einem Flächennormalenvektor des mindestens einen Rührblatts, und einer orthogonal zu einer Rotationsachse der Rührwelle virtuell angeordneten Ebene verstanden werden. Der Anstellwinkel kann hierbei vorzugsweise zwischen größer 0° und kleiner 180° variiert werden. Dadurch können sämtliche Anstellwinkel realisiert werden.

Der Anstellwinkel hat insbesondere einen Einfluss auf ein Strömungsfeld. Daher kann durch die Änderung des Anstellwinkels des mindestens einen Rührblatts in einem Prozess das Strömungsfeld variiert werden.

Durch die bewegliche Anordnung des mindestens einen Rührblatts an dem Rührblattaufnahmeabschnitt, kann, insbesondere in Abhängigkeit von einer Drehrichtung der Rührwelle, der erste Anstellwinkel und/oder der zweite Anstellwinkel eingestellt werden. Dadurch kann insbesondere durch ein Umstellen der Drehrichtung der Rührwelle zwischen zwei Rührertypen umgeschaltet werden. Insbesondere kann die Umstellung in Folge einer auf das mindestens eine Rührblatt wirkenden Kraft, vorzugsweise in Folge einer Drehung der Rührwelle, erfolgen.

Das gemäß dieser Lösung beschriebene Rührwerk ist vorzugsweise so ausgebildet, dass das mindestens eine Rührblatt derart bewegbar an dem Rührblattaufnahmeabschnitt angeordnet ist, dass wenn auf das mindestens eine Rührblatt eine gegen eine erste Drehrichtung der Rührwelle wirkende Kraft wirkt, das mindestens eine Rührblatt in die erste Winkelposition bewegt und/oder in der ersten Winkelposition befestigungsmittellos fixiert wird und/oder das mindestens eine Rührblatt derart bewegbar an dem Rührblattaufnahmeabschnitt angeordnet ist, dass wenn auf das mindestens eine Rührblatt eine gegen eine zweite Drehrichtung der Rührwelle wirkende Kraft wirkt, das mindestens eine Rührblatt in die zweite Winkelposition bewegt und/oder in der zweiten Winkelposition befestigungsmittellos fixiert wird.

Vorzugsweise kann die Rührwelle in die erste Drehrichtung, insbesondere im Uhrzeigersinn, und abwechselnd hierzu in die zweite Drehrichtung, insbesondere gegen den Uhrzeigersinn angetrieben werden. Durch die Drehung der Rührwelle in die erste oder die zweite Drehrichtung kann insbesondere eine gegen diese Drehrichtung wirkende Kraft auf das mindestens eine Rührblatt wirken. Dadurch kann das mindestens eine Rührblatt in die entsprechende Winkelposition gedreht werden. Die entsprechende Kraft kann insbesondere durch das Medium und/oder die Drehung der Rührwelle erzeugt werden.

Insbesondere, wenn eine Bewegung des mindestens einen Rührblatts in eine Richtung begrenzt ist, kann das mindestens eine Rührblatt befestigungsmittellos in einer der Winkelpositionen befestigt werden.

Durch diese Ausgestaltung kann ein besonders einfaches Rührwerk bereitgestellt werden, das zwei verschiedene Rührertypen umfasst.

Das Rührwerk umfasst einen ersten Anschlag , der eine Bewegung des mindestens einen Rührblatts in der ersten Winkelposition in eine erste Bewegungsrichtung begrenzt, und/oder einen zweiten Anschlag, der eine Bewegung des mindestens einen Rührblatts in der zweiten Winkelposition in eine zweite Bewegungsrichtung begrenzt.

Vorzugsweise kann unter der ersten Bewegungsrichtung eine Bewegung von der zweiten Winkelposition in Richtung der ersten Winkelposition verstanden werden. Weiter vorzugsweise kann unter der zweiten Bewegungsrichtung eine Bewegung von der ersten Winkelposition in Richtung der zweiten Winkelposition verstanden werden. Bevorzugt können hierbei die erste Bewegungsrichtung und die zweite Bewegungsrichtung im Wesentlichen entgegengesetzt ausgebildet sein.

Der erste Anschlag und/oder der zweite Anschlag ist an dem Rührblattaufnahmeabschnitt angeordnet oder durch diesen ausgebildet. Beispielsweise kann ein zusätzliches Begrenzungselement vorgesehen sein, dass an dem Rührblattaufnahmeabschnitt angeordnet ist und den ersten Anschlag und/oder den zweiten Anschlag bildet. Beispielsweise könnten auch mehrere zusätzliche Begrenzungselemente vorgesehen sein, die den ersten Anschlag und/oder den zweiten Anschlag und/oder weitere Anschläge, insbesondere einen dritten Anschlag und einen vierten Anschlag, bilden.

Unter einem Anschlag soll demnach jede mögliche Ausgestaltung verstanden werden, die eine Bewegung in die erste Bewegungsrichtung bzw. die zweite Bewegungsrichtung begrenzen kann. Beispielsweise kann eine Fläche oder eine Kante den jeweiligen Anschlag bilden.

Beispielsweise kann das zusätzliche Begrenzungselement vorgesehen sein, dass sowohl eine erste Fläche oder eine erste Kante als auch eine zweite Fläche oder zweite Kante aufweist. Die erste Fläche bzw. die erste Kante kann beispielsweise den ersten Anschlag bilden. Die zweite Fläche bzw. die zweite Kante kann beispielsweise den zweiten Anschlag bilden. Alternativ kann der erste Anschlag und/oder der zweite Anschlag beispielsweise auch durch einen Vorsprung oder einen Steg ausgebildet sein.

Das Rotationselement kann beispielsweise an einer der Rührwelle zugewandten Seite des mindestens einen Rührblatts ausgebildet sein, um das mindestens eine Rührblatt mit dem Rührblattaufnahmeabschnitt zu verbinden. Das Rotationselement kann vorzugsweise zylindrisch ausgebildet sein. Dadurch kann die drehbare Lagerung besonders einfach erzielt werden.

Die Aufnahmeöffnung kann vorzugsweise insbesondere rund, vorzugsweise kreisrund, ausgebildet sein, um das Rotationselement drehbar zu lagern. Insbesondere bevorzugt kann diese runde, vorzugsweise kreisrunde, Aufnahmeöffnung durch den ersten Anschlag und/oder den zweiten Anschlag begrenzt sein, um eine Bewegung in die erste Bewegungsrichtung und/oder in die zweite Bewegungsrichtung zu begrenzen.

Insbesondere bevorzugt kann ein erstes Begrenzungselement vorgesehen sein, das an dem Rührblattaufnahmeabschnitt angeordnet ist und sich in die Aufnahmeöffnung erstreckt, sodass dadurch der erste Anschlag und/oder der zweite Anschlag gebildet werden kann. Insbesondere ist es bevorzugt, dass ein zweites Begrenzungselement an dem Rührblattaufnahmeabschnitt angeordnet ist und sich in die Aufnahmeöffnung erstreckt, sodass dadurch ein dritter Anschlag und/oder ein vierter Anschlag gebildet wird. Beispielsweise kann hierbei die Bewegung des mindestens einen Rührblatts in die erste Bewegungsrichtung durch den ersten Anschlag und den dritten Anschlag begrenzt werden. Insbesondere kann die Bewegung des mindestens einen Rührblatts in die zweite Bewegungsrichtung durch den zweiten Anschlag und den vierten Anschlag begrenzt werden.

Besonders bevorzugt kann das Rotationselement in der Aufnahmeöffnung formschlüssig fixiert, insbesondere verrastet, sein. Dadurch wird eine besonders einfache Anordnung und Befestigung des mindestens einen Rührblatts an dem Rührblattaufnahmeabschnitt ermöglicht. Beispielsweise kann hierbei das Rotationselement ein Rastelement aufweisen, das in der Aufnahmeöffnung verrasten kann.

Ein Rührblatt gemäß dieser Lösung kann vorzugsweise einen Blattbereich aufweisen, der mit dem Rotationselement drehsteif verbunden ist.

Die mindestens eine Aufnahmeöffnung kann hierbei vorzugsweise radial und/oder im Wesentlichen orthogonal zu einer Rotationsachse der Rührwelle ausgebildet sein.

Gemäß einer besonders bevorzugten Ausgestaltung des Rührwerks für einen Bioreaktor ist vorgesehen, dass dieses Rührwerk die Rührwelle und die an der Rührwelle angeordnete Rühreinheit umfasst, wobei die Rühreinheit den Rührblattaufnahmeabschnitt, das mindestens eine Rührblatt und das mit dem Rührblatt drehsteif verbundene Rotationselement umfasst, das zwischen der ersten Drehposition und der zweiten Drehposition drehbar an dem Rührblattaufnahmeabschnitt gelagert ist, wobei das mindestens eine Rührblatt in der ersten Drehposition in dem ersten Anstellwinkel und in der zweiten Drehposition in dem zweiten Anstellwinkel ausgerichtet ist.

Im Betrieb kann somit das Rührblatt entweder in dem ersten Anstellwinkel oder in dem zweiten Anstellwinkel ausgerichtet werden.

Das Rotationselement kann vorzugsweise hierbei derart drehbar an dem Rührblattaufnahmeabschnitt gelagert sein, dass, wenn auf das mindestens eine Rührblatt eine gegen eine erste Drehrichtung der Rührwelle wirkende Kraft wirkt, das Rotationselement in die erste Drehposition bewegt und/oder in der ersten Drehposition befestigungsmittellos fixiert wird, und/oder das Rotationselement derart drehbar an dem Rührblattaufnahmeabschnitt gelagert sein, dass, wenn auf das mindestens eine Rührblatt eine gegen eine zweite Drehrichtung der Rührwelle wirkende Kraft wirkt, das Rotationselement in die zweite Drehposition bewegt und/oder in der zweiten Drehposition befestigungsmittellos fixiert wird.

Das Rotationselement ist vorzugsweise ausgebildet, um durch Drehen um dessen Längsachse in der ersten Drehrichtung von der zweiten Drehposition in die erste Drehposition und durch Drehen um dessen Längsachse in der zweite Drehrichtung von der ersten Drehposition in die zweite Drehposition bewegt zu werden.

Vorzugsweise kann der erste Anschlag an dem Rührblattaufnahmeabschnitt angeordnet sein, gegen den das Rotationselement in der ersten Drehposition anschlagbar ist. Insbesondere bevorzugt kann an dem Rührblattaufnahmeabschnitt der zweite Anschlag angeordnet sein, gegen den das Rotationselement in der zweiten Drehposition anschlagbar ist.

Hierbei kann vorzugsweise das Rotationselement in der ersten Drehposition an dem ersten Anschlag und in der zweiten Drehposition an dem zweiten Anschlag anliegen.

Vorzugsweise kann das Rotationselement eine Querschnittsform aufweisen, die einen ersten Gegenanschlag bildet, der in der ersten Drehposition an dem ersten Anschlag anliegt. Vorzugsweise kann die Querschnittsform des Rotationselements einen zweiten Gegenanschlag bilden, der in der zweiten Drehposition an dem zweiten Anschlag anliegt. Beispielsweise kann das Rotationselement eine im Wesentlichen runde, insbesondere kreisrunde, Querschnittsform mit mindestens einen abgeflachten Abschnitt, vorzugsweise mit zwei abgeflachten Abschnitten, aufweisen. Der jeweilige Gegenanschlag kann vorzugsweise durch einen abgeflachten Abschnitt gebildet werden.

Insbesondere bevorzugt kann die Querschnittsform des Rotationselements einen dritten Gegenanschlag und/oder einen vierten Gegenanschlag bilden.

Vorzugsweise kann der dritte Gegenanschlag in der ersten Drehposition an dem dritten Anschlag anliegen. Insbesondere bevorzugt kann der vierte Gegenanschlag in der zweiten Drehposition an dem vierten Anschlag anliegen.

Beispielsweise kann auch der erste Gegenanschlag in der ersten Drehposition an dem ersten Anschlag anliegen und der zweite Gegenanschlag an dem dritten Anschlag anliegen. Vorzugsweise kann in der zweiten Deposition der erste Gegenanschlag an dem zweiten Anschlag und der zweite Gegenanschlag an dem vierten Anschlag anliegen.

Erfindungsgemäß ist vorgesehen, dass die Aufnahmeöffnung den ersten Anschlag und/oder den zweiten Anschlag bildet oder durch den ersten Anschlag und/oder den zweiten Anschlag begrenzt ist.

Vorzugsweise kann eine die Aufnahmeöffnung begrenzende Scheibe an dem Rührblattaufnahmeabschnitt angeordnet sein, die den ersten Anschlag und/oder den zweiten Anschlag bildet. Insbesondere bevorzugt kann die Scheibe an einer Außenseite oder einer Innenseite des Rührblattaufnahmeabschnitts angeordnet sein.

Beispielsweise kann die Aufnahmeöffnung einen Öffnungsquerschnitt und/oder eine Öffnungsquerschnittsform aufweisen, die den ersten Anschlag und/oder den zweiten Anschlag bildet. Insbesondere bevorzugt kann die Aufnahmeöffnung insbesondere eine runde, insbesondere kreisrunde, Öffnungsquerschnittsform aufweisen. Der jeweilige Anschlag kann insbesondere durch einen abgeflachten Bereich der runden, insbesondere kreisrunden, Öffnungsquerschnittsform gebildet werden.

Gemäß einer besonders bevorzugten Ausgestaltung umfasst das Rührwerk eine Führungseinrichtung, die ausgebildet ist, um eine Bewegung des Rührblatts zwischen der ersten Winkelposition und der zweiten Winkelposition zu führen.

Insbesondere bevorzugt kann das Rotationselement ein Führungselement aufweisen, das ausgebildet ist, um eine Drehung des Rotationselements zwischen der ersten Drehposition und der zweiten Drehposition zu führen. Vorzugsweise kann der Rührblattaufnahmeabschnitt ein korrespondierendes Führungsmittel aufweisen, das ausgebildet ist, um mit dem Führungselement des Rotationselements zusammenzuwirken, um die Drehung des Rotationselements zwischen der ersten Drehposition und der zweiten Drehposition zu führen. Vorzugsweise können das Führungselement und das Führungsmittel ein Führelementsystem bilden.

Vorzugsweise kann das Führungselement als ein Vorsprung ausgebildet sein, der radial von einer Oberfläche oder im Wesentlichen axial und/oder axial-radial von einem freien Ende des Rotationselements hervorsteht. Insbesondere bevorzugt kann das korrespondierende Führungsmittel als eine Führungsschiene ausgebildet sein, die an dem Rührblattaufnahmeabschnitt angeordnet oder ausgebildet ist oder durch einen den Innenumfang definierenden Rand der Aufnahmeöffnung gebildet ist, wobei der Vorsprung in der Führungsschiene geführt wird.

Vorzugsweise kann das Führungselement als ein Rastelement, insbesondere ein Rasthaken, ausgebildet sein, der radial oder axial oder radial-axial von einem freien Ende des Rotationselements hervorsteht. Vorzugsweise kann die Aufnahmeöffnung ein korrespondierendes Führungsmittel ausbilden, in das das Führungselement eingreift und verrastet.

Das Führungsmittel und/oder das Führungselement kann hierbei vorzugsweise den ersten Anschlag und/oder den zweiten Anschlag bzw. den ersten Gegenanschlag und/oder den zweiten Gegenanschlag bilden.

Bevorzugt kann das korrespondierende Führungsmittel, insbesondere die Führungsschiene, durch einen von der Aufnahmeöffnung an einer dem mindestens einen Rührblatt abgewandten Seite gebildeten Rand der Aufnahmeöffnung ausgebildet sein, wobei das Führungselement an dem freien Ende des Rotationselements ausgebildet sein kann und im eingesetzten Zustand hinter diesen Rand greift.

Gemäß einer weiter bevorzugten Ausführungsform kann vorgesehen sein, dass der Rührblattaufnahmeabschnitt und die Rührwelle einstückig ausgebildet sind.

Insbesondere kann vorgesehen sein, dass ein an der Rührwelle angeordnetes Mittelstück den Rührblattaufnahmeabschnitt bildet. Vorzugsweise kann das Mittelstück an der Rührwelle befestigt sein. Insbesondere bevorzugt kann das Mittelstück lösbar an der Rührwelle befestigt sein.

Vorzugsweise kann das Mittelstück als ein Vollzylinder oder ein Hohlzylinder oder ein Ring ausgebildet sein.

Die Rühreinheit kann demnach insbesondere fest mit der Rührwelle verbunden oder lösbar verbunden und austauschbar sein.

Vorzugsweise kann das Mittelstück Schnapphaken aufweisen, die ausgebildet sind, um den Rührblattaufnahmeabschnitt an der Rührwelle zu befestigen. Vorzugsweise können sich die Schnapphaken im Wesentlichen axial von einem Rand des Mittelstücks erstrecken und ausgebildet sein, um mit korrespondierenden Schnappmitteln an der Rührwelle in Eingriff gebracht zu werden.

Insbesondere bevorzugt ist das Mittelstück als ein Hohlzylinder mit einer zentralen Zylinderöffnung ausgebildet. Vorzugsweise kann die zentrale Zylinderöffnung ausgebildet sein, um den Rührblattaufnahmeabschnitt an der Rührwelle anzuordnen und/oder zu befestigen.

Vorzugsweise kann das Mittelstück als ein Ring mit einer zentralen Ringöffnung ausgebildet sein. Vorzugsweise kann die zentrale Ringöffnung ausgebildet sein, um den Rührblattaufnahmeabschnitt an der Rührwelle anzuordnen und/oder zu befestigen.

Insbesondere bevorzugt kann somit das Rührwerk als eine Einheit bereitgestellt werden oder voneinander lösbare Komponenten, insbesondere die Rührwelle und die Rühreinheit und/oder den Rührblattaufnahmeabschnitt und/oder das Rührblatt, umfassen. Die voneinander lösbaren Komponenten können somit vorzugsweise bedarfsorientiert zusammengesetzt und eingesetzt werden.

Gemäß einer weiter bevorzugten Ausführungsform ist vorgesehen, dass der erste Anstellwinkel kleiner 90° oder 90° zu einer orthogonal zu einer Rotationsachse der Rührwelle virtuell angeordneten Ebene ist und/oder der zweite Anstellwinkel 90° oder größer als 90° und kleiner als 180° zu der orthogonal zu der Rotationsachse der Rührwelle virtuell angeordneten Ebene ist.

Besonders bevorzugt ist, wenn der erste Anstellwinkel oder der zweite Anstellwinkel im Wesentlichen 90° zu einer orthogonal zu einer Rotationsachse der Rührwelle virtuell angeordneten Ebene ist und der jeweils andere Anstellwinkel von 90° abweicht und beispielsweise 60° beträgt. Dadurch kann insbesondere umgesetzt werden, dass das Rührwerk einen Scheibenrührer und einen Schrägblattrührer umfasst und somit unterschiedliche Rühreigenschaften umsetzen kann.

Auch Ausführungsformen, in denen das Rührwerk in beiden Winkelpositionen Schrägblattrührer aufweist sind möglich. Beispielsweise kann einer der Anstellwinkel 30° und der andere Anstellwinkel 45° betragen.

Bevorzugt ist vorgesehen, dass der erste Anstellwinkel und der zweite Anstellwinkel unterschiedliche Winkelpositionen definieren.

Weiter ist bevorzugt, dass die Rührwelle und/oder die Rühreinheit zumindest teilweise aus Kunststoff ausgebildet sind oder Kunststoff umfassen oder aus Edelstahl ausgebildet sind oder Edelstahl umfassen. Alternativ können weitere oder alternative Materialien verwendet werden, die Anforderungen für den Einsatz im Bioreaktor erfüllen. Vorzugsweise kann die Rührwelle und/oder die Rühreinheit zumindest teilweise im Spritzgussverfahren oder in einem additiven Herstellungsverfahren hergestellt werden. Beispielsweise kann hierbei die Rührwelle und/oder die Rühreinheit mittels 3D-Druck hergestellt werden.

Vorzugsweise kann die Rührwelle und/oder der Rührblattaufnahmeabschnitt und/oder das Rührblatt und/oder gegebenenfalls das Rotationselement aus Kunststoff ausgebildet sein oder Kunststoff umfassen oder aus Edelstahl ausgebildet sein oder Edelstahl umfassen und/oder im Spritzgussverfahren oder in einem additiven Herstellungsverfahren hergestellt sein.

Besonders bevorzugt ist vorgesehen, dass das Rührwerk mindestens einen von der Rühreinheit axial beabstandeten an der Rührwelle angeordneten Scheibenrührer umfasst.

Vorzugsweise kann das Rührwerk mindestens einen von der Rühreinheit axial beabstandeten an der Rührwelle angeordneten Schrägblattrührer umfassen.

Insbesondere bevorzugt kann das Rührwerk mindestens eine von der Rühreinheit axial beabstandete weitere an der Rührwelle angeordnete Rühreinheit mit einem weiteren Rührblattaufnahmeabschnitt und mindestens einem weiteren Rührblatt umfassen, das an dem weiteren Rührblattaufnahmeabschnitt angeordnet ist und zwischen zwei unterschiedlichen Winkelposition bewegbar ist, in denen das mindestens eine weitere Rührblatt in unterschiedlichen Anstellwinkeln ausgerichtet ist.

Die weitere an der Rührwelle angeordnete Rühreinheit kann vorzugsweise insbesondere im Wesentlichen identisch zu der vorstehend beschriebenen Rührwelle ausgebildet sein. Vorzugsweise kann die weitere Rühreinheit unterschiedliche Anstellwinkel umsetzen, sodass das mindestens eine weitere Rührblatt in einen dritten Anstellwinkel und einen vierten Anstellwinkel ausrichtbar ist. Alternativ kann zumindest ein Anstellwinkel identisch mit dem ersten Anstellwinkel und/oder dem zweiten Anstellwinkel sein.

Insbesondere bevorzugt kann die weitere Rühreinheit ein Rührblatt mit einem drehsteifen Rotationselement umfassen, das zwischen zwei Drehpositionen, in denen das mindestens eine weitere Rührblatt in unterschiedlichen Anstellwinkel ausgerichtet ist, drehbar an dem weiteren Rührblattaufnahmeabschnitt gelagert ist.

Insbesondere bevorzugt kann die mindestens eine weiterer Rühreinheit und/oder der mindestens eine Scheibenrührer und/oder der mindestens eine Schrägblattrührer derart axial beabstandet von der Rühreinheit angeordnet sein, dass die mindestens eine weiterer Rühreinheit und/oder der mindestens eine Scheibenrührer und/oder der mindestens eine Schrägblattrührer erst ab einer definierten Füllhöhe eine Auswirkung auf die Strömung im Medium hat. Auf diese Art und Weise kann die mindestens eine weiterer Rühreinheit und/oder der mindestens eine Scheibenrührer und/oder der mindestens eine Schrägblattrührer in Abhängigkeit der Füllhöhe zugeschaltet werden. Diese Ausgestaltung ist insbesondere dahingehend vorteilhaft, da sich ein Volumen und somit eine Füllhöhe insbesondere während eines Prozesses verändern kann. Gleichzeitig mit der Änderung des Volumens und der Füllhöhe können sich oftmals auch die erforderlichen Rühreigenschaften ändern.

Insbesondere bevorzugt kann das Rührwerk die mindestens eine weitere Rühreinheit umfassen, dessen mindestens ein Rührblatt zwischen dem ersten Anstellwinkel und/oder dem zweiten Anstellwinkel und/oder einem dritten Anstellwinkel und/oder einem vierten Anstellwinkel bewegbar, insbesondere bewegbar gelagert, ist. Vorzugsweise kann das mindestens eine Rührblatt der weiteren Rühreinheit
- in der ersten Drehposition in dem ersten Anstellwinkel und in der zweiten Drehposition in dem zweiten Anstellwinkel ausgerichtet sein oder
- in der ersten Drehposition in einem dritten Anstellwinkel und in der zweiten Drehposition in einem vierten Anstellwinkel ausgerichtet sein oder
- in der ersten Drehposition in dem zweiten Anstellwinkel und in der zweiten Drehposition in dem ersten Anstellwinkel ausgerichtet sein oder
- in der ersten Drehposition in dem ersten Anstellwinkel und in der zweiten Drehposition in einem dritten Anstellwinkel ausgerichtet sein oder
- in der ersten Drehposition in einem dritten Anstellwinkel und in der zweiten Drehposition in dem ersten Anstellwinkel ausgerichtet sein oder
- in der ersten Drehposition in dem zweiten Anstellwinkel und in der zweiten Drehposition in einem dritten Anstellwinkel ausgerichtet sein oder
- in der ersten Drehposition in einem dritten Anstellwinkel und in der zweiten Drehposition in dem zweiten Anstellwinkel ausgerichtet sein.

Gemäß einem weiteren Aspekt ist eine Rühreinheit zum Aufrüsten einer Rührwelle entsprechend Anspruch 9 vorgesehen, umfassend einen Rührblattaufnahmeabschnitt und mindestens ein Rührblatt das an dem Rührblattaufnahmeabschnitt angeordnet ist und zwischen einer ersten Winkelposition und einer zweiten Winkelposition bewegbar ist, wobei das mindestens eine Rührblatt in der ersten Winkelposition in einem ersten Anstellwinkel und in der zweiten Winkelposition in einem zweiten Anstellwinkel ausgerichtet ist. Dabei umfasst das mindestens eine Rührblatt ein drehsteifes Rotationselement, das zwischen einer ersten Drehposition und einer zweiten Drehposition drehbar an dem Rührblattaufnahmeabschnitt gelagert ist. Das mindestens eine Rührblatt ist in der ersten Drehposition in dem ersten Anstellwinkel und in der zweiten Drehposition in dem zweiten Anstellwinkel ausgerichtet. Der Rührblattaufnahmeabschnitt weist mindestens eine Aufnahmeöffnung auf, in der das Rotationselement zwischen der ersten Drehposition und der zweiten Drehposition drehbar gelagert aufgenommen ist. Die Rühreinheit umfasst weiter einen ersten Anschlag, der eine Bewegung des mindestens einen Rührblatts in der ersten Winkelposition in eine erste Bewegungsrichtung begrenzt, und/oder einen zweiten Anschlag, der eine Bewegung des mindestens einen Rührblatts in der zweiten Winkelposition in eine zweite Bewegungsrichtung begrenzt, wobei vorzugsweise der erste Anschlag und/oder der zweite Anschlag an dem Rührblattaufnahmeabschnitt angeordnet oder durch diesen ausgebildet ist, wobei die Aufnahmeöffnung den ersten Anschlag und/oder den zweiten Anschlag bildet oder durch den ersten Anschlag und/oder den zweiten Anschlag begrenzt ist.

Gemäß einem weiteren Aspekt ist ein Bioreaktor entsprechend Anspruch 10 vorgesehen, umfassend eine Kopfplatte und einen Behälter, wobei die Kopfplatte und der Behälter einen Reaktionsraum einschließen, ein im Reaktionsraum angeordnetes Rührwerk, wie hierin beschrieben, wobei die Rührwelle in einem Lager um eine Rotationsachse drehbar gelagert ist, eine außerhalb des Reaktionsraum angeordnete, mit der Rührwelle koppelbare Antriebseinheit, die ausgebildet ist, um die Rührwelle in eine erste Drehrichtung und eine zweite Drehrichtung anzutreiben.

Die erste Drehrichtung und die zweite Drehrichtung können insbesondere entgegengesetzt sein. Insbesondere kann die erste Drehrichtung eine Drehung der Rührwelle im Uhrzeigersinn definieren. Vorzugsweise kann die entsprechend die zweite Drehrichtung eine Drehung der Rührwelle gegen den Uhrzeigersinn definieren.

Vorzugsweise kann die Kopfplatte eine dem Reaktionsraum zugewandte Innenseite und eine Reaktionsraum abgewandte Außenseite mit mehreren Anschlüssen aufweisen. Insbesondere bevorzugt kann der Bioreaktor ein Einwegbioreaktor sein.

Gemäß einem weiteren Aspekt ist ein Verfahren entsprechend Anspruch 11 zum Herstellen einer Rühreinheit, wie hierin beschrieben, und/oder eines Rührwerks, wie hierin beschrieben, umfassend die Schritte: Bereitstellen eines Rührblattaufnahmeabschnitts, Bereitstellen mindestens eines Rührblatts, Anordnen des mindestens einen Rührblatts an dem Rührblattaufnahmeabschnitt, sodass das mindestens eine Rührblatt zwischen einer ersten Winkelposition und einer zweiten Winkelposition derart bewegbar ist, dass das mindestens eine Rührblatt in der ersten Winkelposition in einem ersten Anstellwinkel und in der zweiten Winkelposition in einem zweiten Anstellwinkel ausgerichtbar ist.

Das Rührblatt kann vorzugsweise an dem Rührblattaufnahmeabschnitt angeordnet, insbesondere im Sinne von daran befestigt oder daran ausgebildet werden. Insbesondere kann das Verfahren umfassen: Bereitstellen einer Rührwelle mit einem Rührblattaufnahmeabschnitt. Vorzugsweise kann ferner vorgesehen sein, dass der Rührblattaufnahmeabschnitt an der Rührwelle angeordnet wird.

Vorzugsweise kann ein drehsteifes Rotationselement an dem Rührblattaufnahmeabschnitt angeordnet werden, insbesondere sodass das Rotationselement zwischen der ersten Drehposition und der zweiten Drehposition derart drehbar gelagert ist, dass das mindestens eine Rührblatt in der ersten Drehposition in einem ersten Anstellwinkel und in der zweiten Drehposition in einem zweiten Anstellwinkel ausrichtbar ist.

Gemäß einem weiteren Aspekt ist ein Verfahren entsprechend Anspruch 12 zum Herstellen eines Bioreaktors vorgesehen, insbesondere wie hierin beschrieben, umfassend die Schritte: Bereitstellen eines Behälters, Bereitstellen einer Kopfplatte, Bereitstellen eines Rührwerks, wie hierin beschrieben, Anordnen des Rührwerks an der Kopfplatte, Aufsetzen der Kopfplatte auf den Behälter und Verbinden der Kopfplatte mit dem Behälter, sodass ein Reaktionsraum eingeschlossen ist.

Das Verfahren kann vorzugsweise ein Herstellen eines Rührwerks und/oder einer Rühreinheit, insbesondere wie hierin beschrieben, umfassen.

Ein weiterer Schritt des Verfahrens kann beispielsweise ein Bereitstellen einer Antriebseinheit sein. Die Antriebseinheit kann vorzugsweise an der Kopfplatte angeordnet und mit der Rührwelle gekoppelt werden.

Gemäß einem weiteren Aspekt ist ein Verfahren entsprechend Anspruch 13 zum Betreiben eines Bioreaktors vorgesehen, wie hierin beschrieben, umfassend ein Rührwerk, wie hierin beschrieben, und/oder eine Rühreinheit, wie hierin beschrieben, umfassend die Schritte: bedarfsabhängiges Einstellen eines ersten Antriebsparameters, um eine Rührwelle in eine erste Drehrichtung anzutreiben, sodass mindestens ein Rührblatt in eine erste Winkelposition bewegt wird, in der das mindestens eine Rührblatt in einem ersten Anstellwinkel ausgerichtet ist, und/oder bedarfsabhängiges Einstellen eines zweiten Antriebsparameters, um die Rührwelle in eine zweite Drehrichtung anzutreiben, sodass das mindestens eine Rührblatt in eine zweite Winkelposition bewegt wird, in der das mindestens eine Rührblatt in einem zweiten Anstellwinkel ausgerichtet ist, wobei der erste Antriebsparameter und der zweite Antriebsparameter abwechselnd einstellbar sind.

Vorzugsweise kann die erste Drehrichtung eine Drehung im Uhrzeigersinn beschreiben. Vorzugsweise kann die zweite Drehrichtung eine Drehung gegen den Uhrzeigersinn beschreiben.

Insbesondere bevorzugt kann ein Aspekt beispielweise auch eine Verwendung des hierin beschriebenen Rührwerks und/oder der hierin beschriebenen Rühreinheit sein zum bedarfsabhängiges Einstellen eines ersten Antriebsparameters, um eine Rührwelle in eine erste Drehrichtung anzutreiben, sodass mindestens ein Rührblatt in eine erste Winkelposition bewegt wird, in der das mindestens eine Rührblatt in einem ersten Anstellwinkel ausgerichtet ist, und/oder zum bedarfsabhängiges Einstellen eines zweiten Antriebsparameters, um die Rührwelle in eine zweite Drehrichtung anzutreiben, sodass das mindestens eine Rührblatt in eine zweite Winkelposition bewegt wird, in der das mindestens eine Rührblatt in einem zweiten Anstellwinkel ausgerichtet ist, wobei der erste Antriebsparameter und der zweite Antriebsparameter abwechselnd einstellbar sind.

Zu den Vorteilen, Ausführungsvarianten und Ausführungsdetails dieser weiteren Aspekte und ihrer möglichen Fortbildungen wird auf die vorangegangene Beschreibung zu den entsprechenden Merkmalen verwiesen.

Beispielhafte Ausführungsformen werden beispielhaft anhand der beiliegenden Figuren beschrieben. Es zeigen:
- Fig. 1:: eine beispielhafte Darstellung einer Rühreinheit;
- Fig. 2:: eine beispielhafte Darstellung eines Rührwerks;
- Fig. 3:: eine beispielhafte Darstellung einer Rühreinheit;
- Fig. 4:: eine beispielhafte Schnittdarstellung eines Rotorblattaufnahmeabschnitts;
- Fig. 5 :: eine beispielhafte Schnittdarstellung einer Rühreinheit;
- Fig. 6:: eine beispielhafte Darstellung eines Bioreaktors;
- Fig. 7:: ein beispielhaftes Verfahren zum Herstellen einer Rühreinheit;
- Fig. 8:: ein beispielhaftes Verfahren zum Herstellen eines Bioreaktors; und
- Fig. 9:: ein beispielhaftes Verfahren zum Betreiben eines Bioreaktors.

Ähnliche oder im Wesentlichen funktionsgleiche Elemente werden in den Figuren mit den gleichen Bezugszeichen bezeichnet.

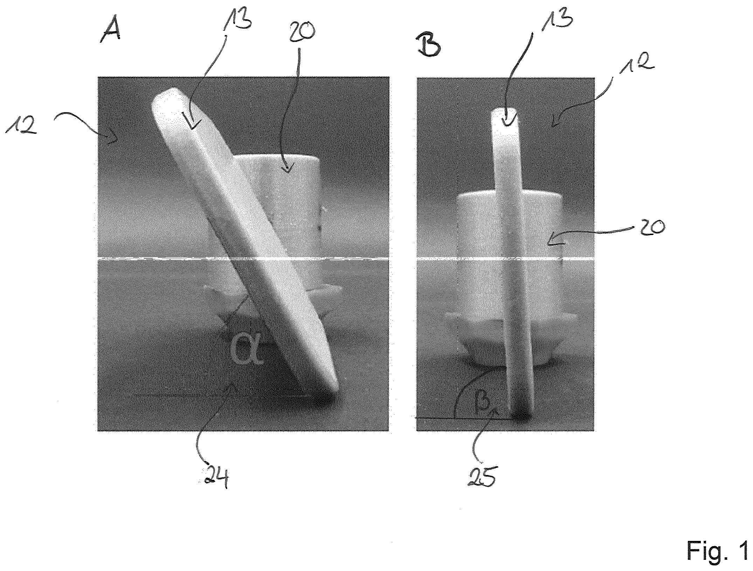

Figur 1 zeigt in A und B jeweils eine beispielhafte Darstellung einer Rühreinheit 12 mit einem Rührblatt 13. Die Rühreinheit 12 weist zudem einen Rührblattaufnahmeabschnitt 20 auf, an dem das Rührblatt 13 angeordnet ist. Es ist insbesondere zu verstehen, dass in A und B dieselbe Rühreinheit 12 dargestellt ist. Das Rührblatt 13 ist zwischen einer ersten Winkelposition, wie in A gezeigt, und einer zweiten Winkelposition, wie in B gezeigt, bewegbar. Das Rührblatt 12 kann insbesondere bewegbar zwischen der ersten Winkelposition und der zweiten Winkelposition an dem Rührblattaufnahmeabschnitt befestigt oder daran ausgebildet sein.

In Figur 1 A ist das Rührblatt 13 in einem ersten Anstellwinkel 24 gezeigt. Der erste Anstellwinkel 24 ist derart gewählt, dass das Rührblatt 13 schräg angeordnet ist und somit die Rühreigenschaften eines Schrägblattrührers erfüllen kann. Der erste Anstellwinkel 24 ist hierbei kleiner 90 ° zu einer orthogonal zu einer Rotationsachse der Rührwelle virtuell angeordneten Ebene.

In Figur 1 B ist das Rührblatt 13 in einem zweiten Anstellwinkel 25 gezeigt. Der zweite Anstellwinkel 25 ist derart gewählt, dass das Rührblatt 13 im Wesentlichen senkrecht angeordnet ist und somit die Rühreigenschaften eines Scheibenrührers erfüllen kann. Der zweite Anstellwinkel 25 ist hierbei in etwa 90° zu einer orthogonal zu einer Rotationsachse der Rührwelle virtuell angeordneten Ebene.

Ein Rührwerk, das zusätzlich zu der in Figur 1 dargestellten Rühreinheit 12 insbesondere auch eine Rührwelle umfasst (in Figur 1 nicht dargestellt), woran die Rühreinheit 12 befestigt oder ausgebildet sein kann, kann insbesondere in einem Bioreaktor eingesetzt werden. Hierbei kann vorzugsweise die Rührwelle mit einer Antriebseinheitdes Bioreaktors gekoppelt werden, um die Rührwelle insbesondere in eine erste Drehrichtung und/oder in eine zweite Drehrichtung antreiben zu können. Die erste Drehrichtung und die zweite Drehrichtung können insbesondere entgegengesetzt, vorzugsweise im Uhrzeigersinn und gegen den Uhrzeigersinn sein. Durch die Drehung der Rührwelle im Betrieb, insbesondere bei Vorhandensein eines Mediums in einem Behälter des Bioreaktors, wirkt eine Kraft entgegengesetzt zu der jeweiligen Drehrichtung auf das Rührblatt 13.

Das Rührblatt 13 ist hierbei insbesondere derart bewegbar an dem Rührblattaufnahmeabschnitt 20 angeordnet, dass wenn auf das Rührblatt 13 eine gegen eine erste Drehrichtung der Rührwelle 11 wirkende Kraft wirkt, das mindestens eine Rührblatt 13 in die erste Winkelposition bewegt wird. Durch das Drehen der Rührwelle kann zudem insbesondere das Rührblatt 13 in der ersten Winkelposition befestigungsmittellos fixiert werden.

Insbesondere ist das Rührblatt 13 derart bewegbar an dem Rührblattaufnahmeabschnitt 20 angeordnet, dass wenn auf das mindestens eine Rührblatt 13 eine gegen eine zweite Drehrichtung der Rührwelle 11 wirkende Kraft wirkt, das mindestens eine Rührblatt 13 in die zweite Winkelposition bewegt wird. Durch das Drehen der Rührwelle kann zudem insbesondere das Rührblatt 13 in der zweiten Winkelposition befestigungsmittellos fixiert werden.

In der hier gezeigten Figur sowie einer Reihe weiterer Figuren ist beispielhaft eine Rühreinheit 12 mit einem einzigen Rührblatt 13 dargestellt, um die strukturellen und funktionellen Merkmale des Rührwerks hervorzuheben. Insbesondere ist die Ausgestaltung der Rühreinheit jedoch nicht auf das gezeigte Beispiel beschränkt. Vielmehr kann verstanden werden, dass die Rühreinheit 12 insgesamt zwei, drei oder mehrere Rührblätter 13 aufweist. Besonders bevorzugt sind insgesamt sechs Rührblätter 13 vorgesehen. Es ist bevorzugte, dass sämtliche Rührblätter 13 der Rühreinheit 12 derart an dem Rührblattaufnahmeabschnitt angeordnet sind, dass diese zwischen der ersten Winkelposition und der zweiten Winkelposition bewegt werden können.

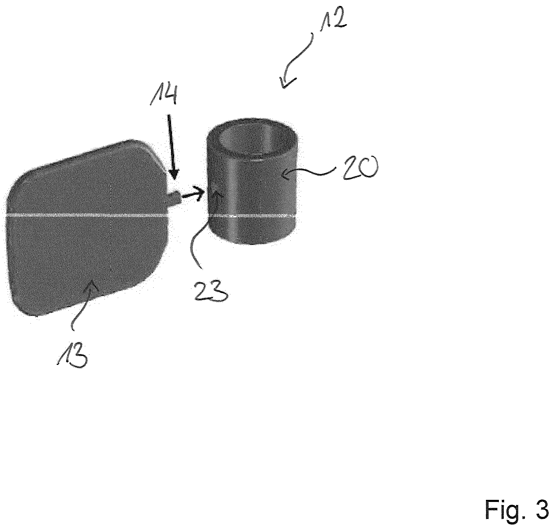

Die Bewegung des Rührblatts 13 zwischen der ersten und der zweiten Winkelposition kann insbesondere in der jeweiligen Richtung begrenzt und/oder geführt sein, um das Einstellen des entsprechenden Anstellwinkels, insbesondere in Abhängigkeit der Drehrichtung der Rührwelle, zu ermöglichen. Hierzu kann beispielsweise ein Anschlagsystem, wie in den Figuren 3 bis 5 beschrieben wird, vorgesehen sein. Alternativ oder ergänzend kann ein Führungssystem vorgesehen sein.

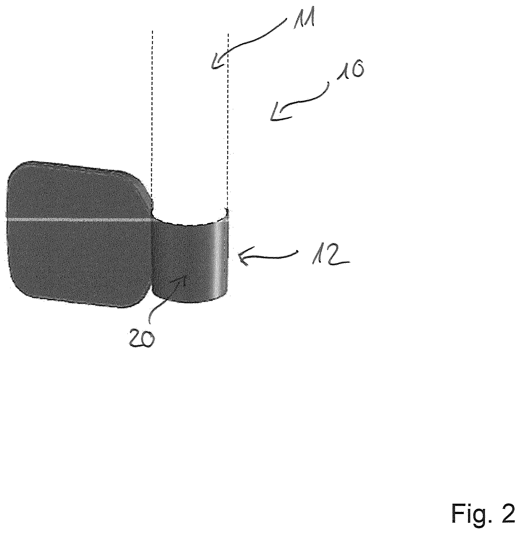

Figur 2 zeigt beispielhaft einen Ausschnitt eines Rührwerks 10 mit einer Rührwelle 11 und einer Rühreinheit 12, die insbesondere wie zuvor beschrieben ausgebildet sein kann. Der Rührblattaufnahmeabschnitt 20 der Rühreinheit 12 kann beispielsweise ein Bestandteil der Rührwelle 11 sein. Alternativ kann die Rühreinheit 12 ein zusätzliches, insbesondere von der Rührwelle 11 trennbares, Element sein. Der Rührblattaufnahmeabschnitt kann hierbei an der Rührwelle befestigt sein. Beispielsweise kann der Rührblattaufnahmeabschnitt 20 als ein Ring oder ein Vollzylinder oder ein Hohlzylinder und ausgebildet sein, um an der Rührwelle 11 befestigt zu werden. Beispielsweise kann der Rührblattaufnahmeabschnitt 20 an einem der Antriebseinheit abgewandten Ende der Rührwelle angeordnet werden. Insbesondere bevorzugt können beispielsweise Schnapphaken vorgesehen sein, die ein Verrasten mit der Rührwelle 11 ermöglichen.

Figur 3 zeigt eine Explosionsdarstellung einer beispielhaften Ausführungsvariante einer Rühreinheit 12. Die Rühreinheit 12 umfasst hierbei einen Rotorblattaufnahmeabschnitt 20, der als hohlzylindrisches Mittelstück ausgebildet ist.

Das Rührblatt 13 umfasst einen Blattbereich und ein drehsteifes Rotationselement 14. Das Rotationselement 14 ist ausgebildet, um in eine Aufnahmeöffnung 23 des Rotorblattaufnahmeabschnitts 20 aufgenommen zu werden. Insbesondere kann das Rotationselement 14 hierbei drehbar gelagert in der Aufnahmeöffnung 23 befestigt werden. Nicht dargestellt sind in dieser Figur insbesondere Befestigungsmittel, wie beispielsweise Schnapphaken, um die Rühreinheit 12 bzw. den Rotorblattaufnahmeabschnitt 20 an einer Rührwelle (nicht gezeigt) befestigen zu können.

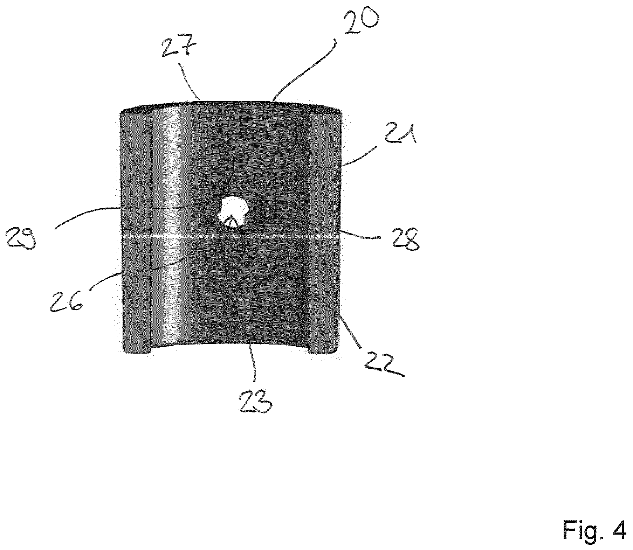

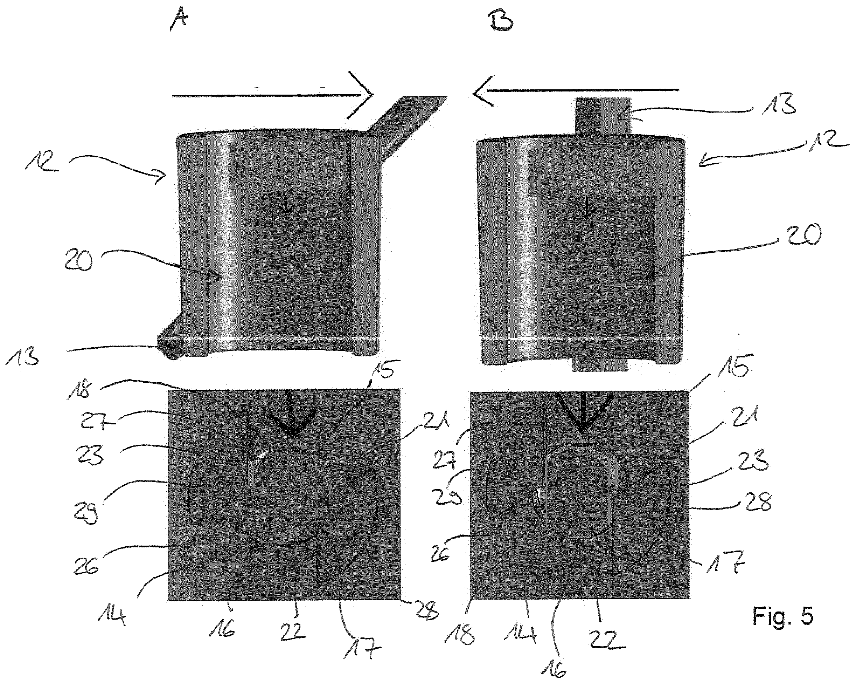

In Figur 4 und in Figur 5 A bzw. B ist eine Schnittdarstellung einer beispielhaften Ausführungsvariante eines Rotorblattaufnahmeabschnitts 20 gezeigt. Sofern dieser Rotorblattaufnahmeschnitt 20 Teil einer Rührwelle ist, kann insbesondere verstanden werden, dass Figur 3 eine Schnittdarstellung eines Abschnitts einer Rührwelle zeigt. Der Rotorblattaufnahmeabschnitt 20 ist hierbei hohlzylindrisch ausgebildet und weist eine Aufnahmeöffnung 23 auf, in die ein Rotationselement aufgenommen werden kann.

In dem hier gezeigten Beispiel ist ein erstes Begrenzungselement 28 und ein zweites Begrenzungselement 29 an einem Innenumfang des Rotorblattaufnahmeabschnitts 20 angeordnet. Das erste Begrenzungselement 28 und das zweite Begrenzungselement 29 ragen in einen Bereich der Aufnahmeöffnung 23, sodass diese begrenzt wird. Das erste Begrenzungselement 28 weist hierbei einen ersten Anschlag 21 und einen zweiten Anschlag 22 auf, die in dem hier gezeigten Beispiel durch Anschlagsflächen des ersten Begrenzungselements 28 gebildet werden. Das zweite Begrenzungselement 29 weist einen dritten Anschlag 26 und einen vierten Anschlag 27 auf, die in dem hier gezeigten Beispiel durch Anschlagsflächen des zweiten Begrenzungselements 29 gebildet werden.

Durch den ersten Anschlag 21 und den dritten Anschlag 26 kann eine Drehbewegung eines in der Aufnahmeöffnung 23 aufgenommenen Rotationselements 14 in einer ersten Drehbewegung beschränkt werden. Entsprechend kann durch den zweiten Anschlag 22 und den vierten Anschlag 27 eine Drehbewegung des in der Aufnahmeöffnung 23 aufgenommenen Rotationselements 14 in einer der ersten Drehbewegung entgegengesetzten zweiten Drehbewegung beschränkt werden.

Durch die in Figur 4 und in Figur 5 A bzw. B dargestellten Begrenzungselemente 28, 29 kann ein Anschlagsystem bereitgestellt werden. Durch ein derartiges Anschlagsystem kann insbesondere das Ausrichten der Rührblätter in bestimmten Anstellwinkel ermöglicht werden. Darüber hinaus wird dadurch die befestigungsmittellose Befestigung der Rührblätter in dem entsprechenden Anstellwinkel ermöglicht. Dadurch wird eine besonders einfache Ausgestaltung der Rühreinheit bereitgestellt.

Beispielsweise kann gemäß einer nicht dargestellten Ausführungsvariante auch lediglich das erste Begrenzungselement 28 oder das zweite Begrenzungselement 29 vorgesehen sein, um die Bewegung des Rotationselements entsprechend in den Drehrichtungen zu beschränken.

Figur 5 zeigt in A und B jeweils eine beispielhafte Darstellung einer Rühreinheit 12 mit einem Rührblatt 13. Es ist insbesondere zu verstehen, dass in A und B dieselbe Rühreinheit 12 dargestellt ist. In Figur 5 A bzw. 5 B ist zusätzlich jeweils eine mit einem Pfeil gekennzeichnete Detailansicht der Rühreinheit gezeigt. Das Rührblatt 13 ist zwischen einer ersten Winkelposition, wie in A gezeigt, und einer zweiten Winkelposition, wie in B gezeigt, bewegbar. Um diese Bewegung zu ermöglichen weist das Rührblatt 13 ein Rotationselement 14 auf, das in der Aufnahmeöffnung 23 drehbar gelagert ist. Zur Befestigung des Rotationselements 14 in der Aufnahmeöffnung 23 weist das Rotationselement ein erstes Rastelement 15 und ein zweites Rastelement 16 auf, die in der Aufnahmeöffnung 23 verrastet sind. In dem hier gezeigten Beispiel sind die Rastelemente 15, 16 an einem einem Blattbereich des Rührblatts 13 abgewandten Ende des Rotationselements 14 ausgebildet und mit einem Rand der Aufnahmeöffnung 23 in Eingriff gebracht.

Das Rotationselement 14 weist eine Querschnittsform mit einem ersten Gegenanschlag 17 und einem zweiten Gegenanschlag 18 auf, die in Abhängigkeit der entsprechenden Drehposition an den Anschlägen 21, 22, 26, 27 anliegen können.

In Figur 5 A wirkt auf das Rührblatt 13 in Abhängigkeit der Drehrichtung der Rührwelle und der daraus resultierenden Strömungsrichtung eines Mediums eine Kraft, die in der Figur mit einem Pfeil gekennzeichnet ist. Dadurch wird das Rührblatt in die erste Winkelposition bewegt und mittels eines Anschlagsystems in dieser ersten Winkelposition befestigungsmittellos fixiert.

Der erste Gegenanschlag 17 liegt, wie in Figur 5 A gezeigt, in der ersten Drehposition an dem ersten Anschlag 21 an. In dieser ersten Drehposition liegt zudem der zweite Gegenanschlag 18 an dem dritten Anschlag 26 an.

In der zweiten Drehposition, wie in Figur 5 B gezeigt, liegt der erste Gegenanschlag 17 an dem zweiten Anschlag 22 und der zweite Gegenanschlag 18 an dem vierten Anschlag 27 an.

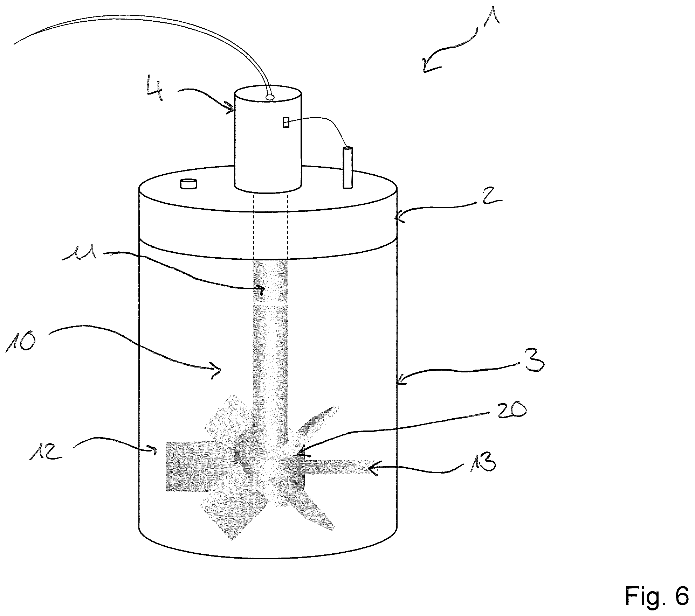

Figur 6 zeigt einen beispielhaften Bioreaktor 1 mit einem Behälter 3 und einer Kopfplatte 2. Die Kopfplatte 2 verschließt in dem gezeigten Beispiel den Behälter 3 derart, dass ein Reaktionsraum eingeschlossen wird. Ein Rührwerk 10 ist in dem Reaktionsraum angeordnet und umfasst eine Rührwelle 11 mit einer Rühreinheit 12. Die Rühreinheit 12 weist einen Rührblattaufnahmeabschnitt 20 auf mit sechs daran angeordneten Rührblättern 13. Die Rührblätter 13 sind in dem hier gezeigten Beispiel in einem ersten Anstellwinkel ausgerichtet, sodass die Rühreinheit 12 die Funktion eines Schrägblattrührers erfüllen kann. Die Rührblätter 12 sind auch in dem hier gezeigten Beispiel zwischen einer ersten Winkelposition und einer zweiten Winkelposition bewegbar angeordnet, sodass die Rührblätter in dem ersten Anstellwinkel oder dem zweiten Anstellwinkel, insbesondere in Abhängigkeit der Drehrichtung der Rührwelle 11, ausgerichtet werden können. Durch die Drehung der Rührwelle 11 können die Rührblätter 12 auch in der jeweiligen Winkelposition insbesondere befestigungsmittellos befestigt werden.

Außerhalb des Reaktionsraums ist zudem eine Antriebseinheit 4 an der Koppelplatte 2 angeordnet. Die Antriebseinheit 4 ist mit der Rührwelle 11 gekoppelt. Dadurch kann die Rührwelle 11 entweder in eine erste Drehrichtung oder in eine zweite Drehrichtung angetrieben werden. Durch diese Ausgestaltung kann insbesondere die Rühreigenschaft in Abhängigkeit der Drehrichtung, insbesondere innerhalb eines kontinuierlichen Prozesses, geändert werden.

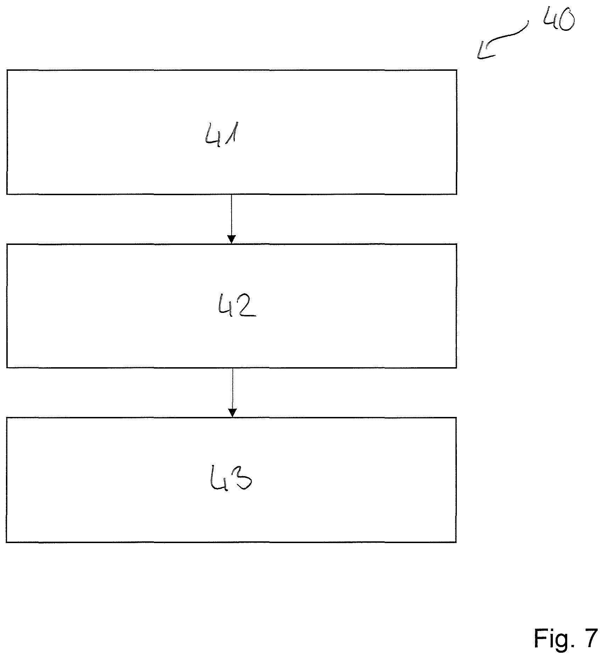

In Figur 7 sind die Verfahrensschritte eines Verfahrens zum Herstellen einer Rühreinheit, wie hierin beschrieben, eines Rührwerks und/oder eines Rührwerks 40 beispielhaft skizziert. Das Verfahren umfasst die Schritte Bereitstellen eines Rührblattaufnahmeabschnitts 41, Bereitstellen mindestens eines Rührblatts 42 und Anordnen des mindestens einen Rührblatts an dem Rührblattaufnahmeabschnitt 43, sodass das mindestens eine Rührblatt zwischen einer ersten Winkelposition und einer zweiten Winkelposition derart bewegbar ist, dass das mindestens eine Rührblatt in der ersten Winkelposition in einem ersten Anstellwinkel und in der zweiten Winkelposition in einem zweiten Anstellwinkel ausgerichtbar ist. Beispielsweise kann das Verfahre zusätzliche Schritte zum Herstellen, insbesondere des Rührwerks, umfassen. Beispielsweise kann das Verfahren ferner den Schritt umfassen: Bereitstellen einer Rührwelle mit einem Rührblattaufnahmeabschnitt.

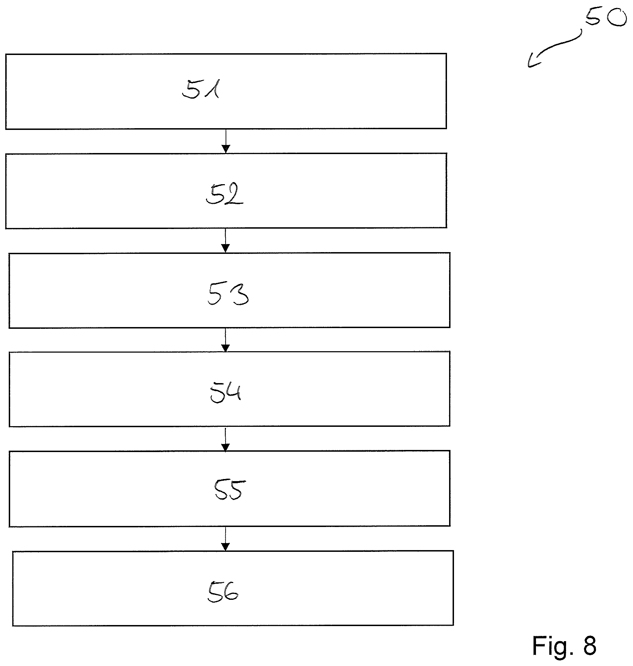

Figur 8 zeigt beispielhaft Verfahrensschritte eines Verfahrens zum Herstellen eines Bioreaktors. Das Verfahren sieht das Bereitstellen eines Behälters 51 und das Bereitstellen einer Kopfplatte 52 vor. In Schritt 53 wird ein Rührwerk wie hierin beschrieben bereitgestellt und in Schritt 54 an der Kopfplatte angeordnet. Schließlich sieht das Verfahren in Schritt 55 das Aufsetzen der Kopfplatte auf den Behälter und in Schritt 56 schließlich das Verbinden der Kopfplatte mit dem Behälter, sodass ein Reaktionsraum eingeschlossen ist, vor.

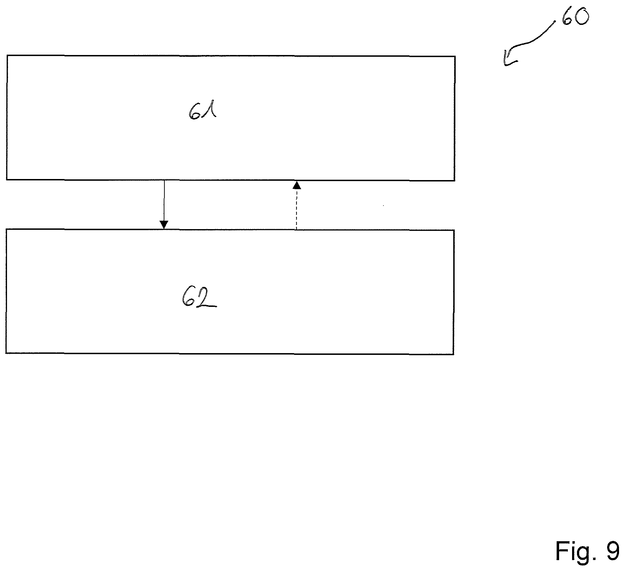

In Figur 9 sind beispielhafte Verfahrensschritte eines Verfahrens zum Betreiben eines Bioreaktors veranschaulicht. In Schritt 61 kann in Abhängigkeit eines Bedarfs, also insbesondere in Abhängigkeit erforderlicher Rühreigenschaften und/oder Eigenschaften eines Mediums, ein erster Antriebsparameter eingestellt werden. Dadurch wird die Rührwelle in eine erste Drehrichtung angetrieben, sodass das mindestens eine Rotorblatt der Rühreinheit des Rührwerks in die erste Winkelposition bewegt wird, in der das mindestens eine Rührblatt in dem ersten Anstellwinkel ausgerichtet ist. Bei Bedarf, also insbesondere in Abhängigkeit erforderlicher Rühreigenschaften und/oder Eigenschaften des Mediums, kann in Schritt 62 ein zweiter Antriebsparameter eingestellt werden, um die Rührwelle in eine zweite Drehrichtung anzutreiben. Dadurch kann das mindestens eine Rührblatt in eine zweite Winkelposition bewegt werden, in der das mindestens eine Rührblatt in dem zweiten Anstellwinkel ausgerichtet ist. Die Schritte 61 und 62 können hierbei insbesondere innerhalb eines Prozesses abwechselnd eingestellt werden. Dadurch können die Rühreigenschaften im Rahmen eines Prozesses angepasst werden.

### Bezugszeichenliste

- 1: Bioreaktor
- 2: Kopfplatte
- 3: Behälter
- 4: Antriebseinheit
- 10: Rührwerk
- 11: Rührwelle
- 12: Rühreinheit
- 13: Rührblatt
- 14: Rotationselement
- 15: erstes Rastelement
- 16: zweites Rastelement
- 17: erster Gegenanschlag
- 18: zweiter Gegenanschlag
- 20: Rührblattaufnahmeabschnitt
- 21: erster Anschlag
- 22: zweiter Anschlag
- 23: Aufnahmeöffnung
- 24: erster Anstellwinkel
- 25: zweiter Anstellwinkel
- 26: dritter Anschlag
- 27: vierter Anschlag
- 28: erstes Begrenzungselement
- 29: zweites Begrenzungselement
- 30: Führungselementsystem
- 31: Führungselement
- 32: Führungsmittel
- 40: Verfahren zum Herstellen einer Rühreinheit und/oder eines Rührwerks
- 41: Bereitstellen eines Rührblattaufnahmeabschnitts
- 42: Bereitstellen mindestens eines Rührblatts
- 43: Anordnen des mindestens einen Rührblatts an dem Rührblattaufnahmeabschnitt
- 50: Verfahren zum Herstellen eines Bioreaktors
- 51: Bereitstellen eines Behälters
- 52: Bereitstellen einer Kopfplatte
- 53: Bereitstellen eines Rührwerks
- 54: Anordnen des Rührwerks an der Kopfplatte
- 55: Aufsetzen der Kopfplatte auf den Behälter
- 56: Verbinden der Kopfplatte mit dem Behälter
- 60: Verfahren zum Betreiben eines Bioreaktors
- 61: bedarfsabhängiges Einstellen eines ersten Antriebsparameters
- 62: bedarfsabhängiges Einstellen eines zweiten Antriebsparameters

## Patentansprüche

1. Rührwerk (10) für einen Bioreaktor (1), umfassend eine Rührwelle (11) mit einer Rühreinheit (12) mit
- einem Rührblattaufnahmeabschnitt (20) und
- mindestens einem Rührblatt (13), das an dem Rührblattaufnahmeabschnitt (20) angeordnet und zwischen einer ersten Winkelposition und einer zweiten Winkelposition bewegbar ist,
wobei das mindestens eine Rührblatt (13) in der ersten Winkelposition in einem ersten Anstellwinkel (24) und in der zweiten Winkelposition in einem zweiten Anstellwinkel (25) ausgerichtet ist, wobei das mindestens eine Rührblatt (13) ein drehsteifes Rotationselement (14) umfasst, das zwischen einer ersten Drehposition und einer zweiten Drehposition drehbar an dem Rührblattaufnahmeabschnitt (20) gelagert ist,
wobei das mindestens eine Rührblatt (13) in der ersten Drehposition in dem ersten Anstellwinkel (24) und in der zweiten Drehposition in dem zweiten Anstellwinkel (25) ausgerichtet ist,
wobei der Rührblattaufnahmeabschnitt (20) mindestens eine Aufnahmeöffnung (23) aufweist, in der das Rotationselement (14) zwischen der ersten Drehposition und der zweiten Drehposition drehbar gelagert aufgenommen ist,
die Rühreinheit weiter umfassend
- einen ersten Anschlag, der eine Bewegung des mindestens einen Rührblatts (13) in der ersten Winkelposition in eine erste Bewegungsrichtung begrenzt, und/oder
- einen zweiten Anschlag, der eine Bewegung des mindestens einen Rührblatts (13) in der zweiten Winkelposition in eine zweite Bewegungsrichtung begrenzt, wobei vorzugsweise der erste Anschlag und/oder der zweite Anschlag an dem Rührblattaufnahmeabschnitt angeordnet oder durch diesen ausgebildet ist, wobei die Aufnahmeöffnung (23) den ersten Anschlag (21) und/oder den zweiten Anschlag (22) bildet oder durch den ersten Anschlag (21) und/oder den zweiten Anschlag (22) begrenzt ist.

2. Rührwerk (10) nach dem vorhergehenden Anspruch 1,
- wobei das mindestens eine Rührblatt (13) derart bewegbar an dem Rührblattaufnahmeabschnitt (20) angeordnet ist, dass wenn auf das mindestens eine Rührblatt (13) eine gegen eine erste Drehrichtung der Rührwelle (11) wirkende Kraft wirkt, das mindestens eine Rührblatt (13) in die erste Winkelposition bewegt und/oder in der ersten Winkelposition befestigungsmittellos fixiert wird, und/oder
- wobei das mindestens eine Rührblatt (13) derart bewegbar an dem Rührblattaufnahmeabschnitt (20) angeordnet ist, dass wenn auf das mindestens eine Rührblatt (13) eine gegen eine zweite Drehrichtung der Rührwelle (11) wirkende Kraft wirkt, das mindestens eine Rührblatt (13) in die zweite Winkelposition bewegt und/oder in der zweiten Winkelposition befestigungsmittellos fixiert wird.

3. Rührwerk (10) nach mindestens einem der vorhergehenden Ansprüche,
wobei das Rotationselement (14) in der Aufnahmeöffnung (23) formschlüssig fixiert, insbesondere verrastet, ist.

4. Rührwerk (10) nach mindestens einem der vorhergehenden Ansprüche, umfassend eine Führungseinrichtung, die ausgebildet ist, um eine Bewegung des Rührblatts zwischen der ersten Winkelposition und der zweiten Winkelposition zu führen.

5. Rührwerk (10) nach mindestens einem der vorhergehenden Ansprüche, wobei
- der Rührblattaufnahmeabschnitt (20) und die Rührwelle (11) einstückig ausgebildet sind oder
- ein an der Rührwelle (11) angeordnetes Mittelstück den Rührblattaufnahmeabschnitt (20) bildet,
wobei vorzugsweise das Mittelstück an der Rührwelle (11) befestigt ist,
wobei vorzugsweise das Mittelstück als ein Vollzylinder oder ein Hohlzylinder oder ein Ring ausgebildet ist.

6. Rührwerk (10) nach mindestens einem der vorhergehenden Ansprüche,
wobei der erste Anstellwinkel (24) kleiner 90° oder 90° zu einer orthogonal zu einer Rotationsachse der Rührwelle (11) virtuell angeordneten Ebene ist und/oder
wobei der zweite Anstellwinkel (25) 90° oder größer als 90° und kleiner als 180° zu der orthogonal zu der Rotationsachse der Rührwelle (11) virtuell angeordneten Ebene ist.

7. Rührwerk (10) nach mindestens einem der vorhergehenden Ansprüche, wobei die Rührwelle (11) und/oder die Rühreinheit (12) zumindest teilweise aus Kunststoff ausgebildet sind oder Kunststoff umfassen oder aus Edelstahl ausgebildet sind oder Edelstahl umfassen,
wobei vorzugsweise die Rührwelle (11) und/oder die Rühreinheit (12) zumindest teilweise im Spritzgussverfahren oder in einem additiven Herstellungsverfahren hergestellt sind.

8. Rührwerk (10) nach mindestens einem der vorhergehenden Ansprüche, umfassend
- mindestens einen von der Rühreinheit (12) axial beabstandeten an der Rührwelle (11) angeordneten Scheibenrührer und/oder
- mindestens einen von der Rühreinheit (12) axial beabstandeten an der Rührwelle (11) angeordneten Schrägblattrührer und/oder
- mindestens eine von der Rühreinheit (12) axial beabstandete weitere an der Rührwelle (11) angeordnete Rühreinheit mit einem weiteren Rührblattaufnahmeabschnitt und mindestens einem weiteren Rührblatt, das an dem weiteren Rührblattaufnahmeabschnitt angeordnet ist und zwischen zwei unterschiedlichen Winkelpositionen bewegbar ist, in denen das mindestens eine weitere Rührblatt in unterschiedlichen Anstellwinkeln ausgerichtet ist.

9. Rühreinheit (12) zum Aufrüsten einer Rührwelle (11), umfassend
- einen Rührblattaufnahmeabschnitt (20) und
- mindestens ein Rührblatt (13), das an dem Rührblattaufnahmeabschnitt (20) angeordnet ist und zwischen einer ersten Winkelposition und einer zweiten Winkelposition bewegbar ist,
wobei das mindestens eine Rührblatt (13) in der ersten Winkelposition in einem ersten Anstellwinkel (24) und in der zweiten Winkelposition in einem zweiten Anstellwinkel (25) ausgerichtet ist, wobei das mindestens eine Rührblatt (13) ein drehsteifes Rotationselement (14) umfasst, das zwischen einer ersten Drehposition und einer zweiten Drehposition drehbar an dem Rührblattaufnahmeabschnitt (20) gelagert ist,
wobei das mindestens eine Rührblatt (13) in der ersten Drehposition in dem ersten Anstellwinkel (24) und in der zweiten Drehposition in dem zweiten Anstellwinkel (25) ausgerichtet ist,
wobei der Rührblattaufnahmeabschnitt (20) mindestens eine Aufnahmeöffnung (23) aufweist, in der das Rotationselement (14) zwischen der ersten Drehposition und der zweiten Drehposition drehbar gelagert aufgenommen ist,
weiter umfassend
- einen ersten Anschlag, der eine Bewegung des mindestens einen Rührblatts (13) in der ersten Winkelposition in eine erste Bewegungsrichtung begrenzt, und/oder
- einen zweiten Anschlag, der eine Bewegung des mindestens einen Rührblatts (13) in der zweiten Winkelposition in eine zweite Bewegungsrichtung begrenzt, wobei vorzugsweise der erste Anschlag und/oder der zweite Anschlag an dem Rührblattaufnahmeabschnitt angeordnet oder durch diesen ausgebildet ist, wobei die Aufnahmeöffnung (23) den ersten Anschlag (21) und/oder den zweiten Anschlag (22) bildet oder durch den ersten Anschlag (21) und/oder den zweiten Anschlag (22) begrenzt ist.

10. Bioreaktor (1), umfassend
- eine Kopfplatte (2) und einen Behälter (3),
wobei die Kopfplatte (2) und der Behälter (3) einen Reaktionsraum einschließen,
- ein im Reaktionsraum angeordnetes Rührwerk (10) nach mindestens einem der vorhergehenden Ansprüche 1-8,
wobei die Rührwelle (11) in einem Lager um eine Rotationsachse drehbar gelagert ist,
- eine außerhalb des Reaktionsraum angeordnete, mit der Rührwelle (11) koppelbare Antriebseinheit (4), die ausgebildet ist, um die Rührwelle (11) in eine erste Drehrichtung und eine zweite Drehrichtung anzutreiben.

11. Verfahren zum Herstellen einer Rühreinheit nach Anspruch 9, und/oder eines Rührwerks (40) nach mindestens einem der Ansprüche 1 bis 8, umfassend die Schritte:
- Bereitstellen eines Rührblattaufnahmeabschnitts (41),
- Bereitstellen mindestens eines Rührblatts (42),
- Anordnen des mindestens einen Rührblatts an dem Rührblattaufnahmeabschnitt (43), sodass das mindestens eine Rührblatt zwischen einer ersten Winkelposition und einer zweiten Winkelposition derart bewegbar ist, dass das mindestens eine Rührblatt in der ersten Winkelposition in einem ersten Anstellwinkel und in der zweiten Winkelposition in einem zweiten Anstellwinkel ausgerichtbar ist.

12. Verfahren zum Herstellen eines Bioreaktors (50), insbesondere nach dem vorhergehenden Anspruch 10, umfassend die Schritte:
- Bereitstellen eines Behälters (51),
- Bereitstellen einer Kopfplatte (52),
- Bereitstellen eines Rührwerks nach einem der Ansprüche 1 bis 8 (53),
- Anordnen des Rührwerks an der Kopfplatte (54),
- Aufsetzen der Kopfplatte auf den Behälter (55) und
- Verbinden der Kopfplatte mit dem Behälter (56), sodass ein Reaktionsraum eingeschlossen ist.

13. Verfahren zum Betreiben eines Bioreaktors (60) nach dem vorhergehenden Anspruch 10, umfassend ein Rührwerk nach mindestens einem der Ansprüche 1 bis 8, und/oder eine Rühreinheit nach Anspruch 9, umfassend die Schritte:
- bedarfsabhängiges Einstellen eines ersten Antriebsparameters (61), um eine Rührwelle in eine erste Drehrichtung anzutreiben, sodass mindestens ein Rührblatt in eine erste Winkelposition bewegt wird, in der das mindestens eine Rührblatt in einem ersten Anstellwinkel ausgerichtet ist, und/oder
- bedarfsabhängiges Einstellen eines zweiten Antriebsparameters (62), um die Rührwelle in eine zweite Drehrichtung anzutreiben, sodass das mindestens eine Rührblatt in eine zweite Winkelposition bewegt wird, in der das mindestens eine Rührblatt in einem zweiten Anstellwinkel ausgerichtet ist,
wobei der erste Antriebsparameter und der zweite Antriebsparameter abwechselnd einstellbar sind.

## Claims

1. Agitator (10) for a bioreactor (1), comprising an agitator shaft (11) with a stirring unit (12) having
- a stirrer blade mounting section (20) and
- at least one agitator blade (13) arranged on the agitator blade mounting section (20) and movable between a first angular position and a second angular position,
wherein the at least one agitator blade (13) is positioned at a first angle (24) in the first angular position and at a second angle
angle (25), wherein the at least one stirring blade (13) comprises a rotationally rigid rotational element (14) that is rotatably mounted on the stirring blade mounting section (20) between a first rotational position and a second rotational position,
wherein the at least one stirring blade (13) is aligned in the first angle (24) and, in the second rotational position, is aligned in the second angle (25),
wherein the stirrer blade mounting section (20) has at least one mounting opening (23) in which the rotary element (14) is rotatably mounted between the first rotational position and the second rotational position,
the stirring unit further comprising
- a first stop that limits movement of the at least one stirring blade (13) in the first angular position in a first direction of movement, and/or
- a second stop that limits movement of the at least one stirring blade (13) in the second angular position in a second direction of movement, wherein preferably the first stop and/or the second stop is arranged on the stirrer blade receiving portion or is formed by it, wherein the receiving opening (23) forms the first stop (21) and/or the second stop (22) or is formed by the first stop (21) and/or the second
stop (22).

2. Agitator (10) according to the preceding claim 1,
- wherein the at least one stirring blade (13) is movably arranged on the stirring blade mounting section (20) such that when a force acting against a first direction of rotation of the stirring shaft (11) is applied to the at least one stirring blade (13)
force acts on the at least one agitator blade (13), the at least one agitator blade (13) moves into the first angular position and/or is fixed in the first angular position without fasteners, and/or
- wherein the at least one stirring blade (13) is movably arranged on the stirring blade mounting section (20) such that when a force acting against a first direction of rotation of the stirring shaft (11) is applied to the at least one stirring blade (13), the at least one stirring blade (13) moves into the second angular position and/or is fixed in the second angular position without fasteners.

3. Agitator (10) according to at least one of the preceding claims,
wherein the rotating element (14) is fixed in the receiving opening (23) by positive locking,
in particular, is latched.

4. Agitator (10) according to at least one of the preceding claims, comprising a guide device configured to guide movement of the agitator blade between the first angular position and the second angular position.

5. Agitator (10) according to at least one of the preceding claims, wherein
- the stirrer blade mounting section (20) and the stirrer shaft (11) are formed as a single piece, or
- a central piece arranged on the agitator shaft (11) forms the agitator blade mounting section (20),
wherein the intermediate piece is preferably secured to the stirring shaft (11), wherein the intermediate piece is preferably formed as a solid cylinder, a hollow cylinder, or a ring.

6. Agitator (10) according to at least one of the preceding claims,
wherein the first angle of attack (24) is less than 90° or 90° relative to a plane virtually arranged orthogonal to a rotational axis of the stirring shaft (11) and/or
wherein the second angle of attack (25) is 90° or greater than 90° and less than 180° relative to the plane virtually arranged orthogonal to the axis of rotation of the stirrer shaft (11).

7. Agitator (10) according to at least one of the preceding claims, wherein the agitator shaft (11) and/or the agitator unit (12) are at least partially made of or comprise plastic, or are made of or comprise stainless steel,
wherein preferably the stirring shaft (11) and/or the stirring unit (12) are at least partially
by injection molding or an additive manufacturing process.

8. Agitator (10) according to at least one of the preceding claims, comprising
- at least one disc agitator arranged on the agitator shaft (11) and axially spaced from the agitator unit (12), and/or
- at least one inclined-blade agitator arranged on the agitator shaft (11) and axially spaced from the agitator unit (12), and/or
- at least one further stirring unit arranged on the stirring shaft (11) and axially spaced from the stirring unit (12), having a further stirring blade mounting section and at least one further stirring blade mounted on the further stirring blade mounting section and is movable between two different angular positions in which the at least one additional stirring blade is aligned at different angles of attack.

9. Stirring unit (12) for retrofitting a stirring shaft (11), comprising
- a paddle mounting section (20) and
- at least one agitator blade (13) arranged on the agitator blade mounting section (20) and movable between a first angular position and a second angular position,
wherein the at least one stirring blade (13) is aligned at a first angle (24) in the first angular position and at a second angle (25) in the second angular position, wherein the at least one stirring blade (13) comprises a rotationally rigid rotational element (14) that is movable between a first rotational position and a second rotational position,
wherein the at least one stirring blade (13) is aligned in the first angle position at the first angle (24) and in the second angle position at the second angle (25),
wherein the impeller blade mounting section (20) has at least one mounting opening (23) in which the rotary element (14) is rotatably mounted between the first rotational position and the second rotational position,
further comprising
- a first stop that prevents movement of the at least one stirring blade (13) in the first angular position in a first direction of movement, and/or
- a second stop that limits movement of the at least one stirring blade (13) in the second angular position in a second direction of movement, wherein preferably the first stop and/or the second stop is provided on the stirrer blade receiving section, wherein the receiving opening (23) forms the first stop (21) and/or the second stop (22) or is limited by the first stop (21) and/or the second stop (22).

10. Bioreactor (1) comprising
- a head plate (2) and a vessel (3),
wherein the head plate (2) and the vessel (3) enclose a reaction chamber,
- a stirrer (10) arranged in the reaction chamber according to at least one of the preceding claims 1-8,
wherein the stirrer shaft (11) is rotatably mounted in a bearing about an axis of rotation,
- a drive shaft (12) arranged outside the reaction chamber and coupled to the stirrer shaft (11),

11. A method for manufacturing a stirring unit according to claim 9, and/or a stirrer (40) according to at least one of claims 1 to 8, comprising the steps of:
- providing a stirrer blade mounting section (41),
- providing at least one stirring blade (42),
- mounting the at least one stirring blade on the stirring blade mounting section (43) such that the at least one stirring blade is movable between a first angular position and a second angular position in such a way that the at least one stirring blade, in the first angular position, is in a first angle of attack and in the second angular position is in a second angle of attack.

12. A method for manufacturing a bioreactor (50), in particular according to the preceding claim 10, comprising the steps of:
- providing a container (51),
- providing a head plate (52),
- providing an agitator according to one of claims 1 through 8 (53),
- mounting the agitator on the head plate (54),
- placing the head plate on the container (55), and
- connecting the head plate to the container (56) so that a reaction chamber is formed.

13. A method for operating a bioreactor (60) according to the preceding claim 10, comprising a stirrer according to at least one of claims 1 to 8, and/or a stirring unit according to claim 9, comprising the steps of:
- adjusting a first drive parameter (61) as needed to stirring shaft in a first direction of rotation so that at least one stirring blade is moved to a first angular position in which the at least one stirring blade is aligned at a first angle of attack, and/or
- demand-dependent adjustment of a second drive parameter (62) to drive the stirring shaft in a second direction of rotation, such that the at least at least one stirring blade is moved to a second angular position in which the at least one stirring blade is aligned at a second angle of attack,
wherein the first drive parameter and the second drive parameter are adjustable alternately.

## Revendications

1. Agitateur (10) pour un bioréacteur (1), comprenant un arbre d'agitation (11) avec une
unité d'agitation (12) comportant
- une section de montage de pales d'agitation (20) et
- au moins une pale d'agitateur (13) disposée sur la section de montage de pale d'agitateur (20) et mobile entre une première position angulaire et une deuxième position angulaire,
dans lequel la au moins une pale d'agitateur (13) est positionnée selon un premier angle (24) dans la première position angulaire et selon un deuxième angle
(25), dans laquelle la au moins une pale d'agitation (13) comprend un élément rotatif (14) rigide en rotation qui est monté de manière rotative sur la section de montage de pale d'agitation (20) entre une première position de rotation et une deuxième position de rotation,
dans lequel la au moins une pale d'agitation (13) est alignée dans la première angle (24) et, dans la deuxième position de rotation, est alignée selon le deuxième angle (25),
dans lequel la section de montage de la pale d'agitation (20) comporte au moins une ouverture de montage (23) dans laquelle l'élément rotatif (14) est monté de manière rotative entre la première position de rotation et la deuxième position de rotation,
l'unité d'agitation comprenant en outre
- une première butée qui limite le mouvement de la au moins une pale d'agitation
(13) dans la première position angulaire dans une première direction de déplacement, et/ou
- une deuxième butée qui limite le mouvement de la au moins une pale d'agitation (13) dans la deuxième position angulaire dans une deuxième direction de déplacement,
dans lequel, de préférence, la première butée et/ou la deuxième butée est disposée sur la partie de réception de la pale d'agitation ou est formée par celle-ci, dans lequel l'ouverture de réception (23) forme la première butée (21) et/ou la deuxième butée (22) ou est formée par la première butée (21) et/ou la deuxième
butée (22).

2. Agitateur (10) selon la revendication 1 précédente,
- dans lequel la au moins une pale d'agitation (13) est disposée de manière mobile sur la section de montage de la pale d'agitation (20) de telle sorte que, lorsqu'une force agissant à l'encontre d'une première direction de rotation de l'arbre d'agitation (11) est appliquée à la au moins une pale d'agitation (13)
force agit sur la au moins une pale d'agitation (13), la au moins une pale d'agitation (13) se déplace vers la première position angulaire et/ou est fixée dans la première position angulaire sans éléments de fixation, et/ou
- dans lequel la au moins une pale d'agitation (13) est disposée de manière mobile sur la section de montage de la pale d'agitation (20) de telle sorte que, lorsqu'une force agissant à l'encontre d'une première direction de rotation de l'arbre d'agitation (11) est appliquée à la au moins une pale d'agitation (13), ladite au moins une pale d'agitation (13) se déplace dans la deuxième position angulaire et/ou est fixée dans la deuxième position angulaire sans éléments de fixation.

3. Agitateur (10) selon au moins l'une des revendications précédentes,
dans lequel l'élément rotatif (14) est fixé dans l'ouverture de réception (23) par un verrouillage par complémentarité de forme,
en particulier, est encliqueté.

4. Agitateur (10) selon au moins l'une des revendications précédentes, comprenant un dispositif de guidage configuré pour guider le mouvement de la pale d'agitation entre la première position angulaire et la deuxième position angulaire.

5. Agitateur (10) selon au moins l'une des revendications précédentes, dans lequel
- la section de montage de la pale d'agitateur (20) et l'arbre d'agitateur (11) sont formés d'une seule pièce, ou
- une pièce centrale disposée sur l'arbre d'agitateur (11) forme la section de montage de la pale d'agitateur (20), dans lequel la pièce intermédiaire est de préférence fixée à l'arbre d'agitation (11),
dans lequel la pièce intermédiaire est de préférence réalisée sous la forme d'un cylindre plein, d'un cylindre creux ou d'un anneau.

6. Agitateur (10) selon au moins l'une des revendications précédentes,
dans lequel le premier angle d'attaque (24) est inférieur à 90° ou égal à 90° par rapport à un plan pratiquement orthogonal à un axe de rotation de l'arbre d'agitation (11) et/ou
dans lequel le deuxième angle d'attaque (25) est de 90° ou supérieur à 90° et inférieur à 180° par rapport au plan pratiquement orthogonal à l'axe de rotation de l'arbre d'agitation (11).

7. Agitateur (10) selon au moins l'une des revendications précédentes, dans lequel l'arbre d'agitation (11) et/ou l'unité d'agitation (12) sont au moins partiellement constitués de ou comprennent du plastique, ou sont constitués de ou comprennent de l'acier inoxydable,
dans lequel, de préférence, l'arbre d'agitation (11) et/ou l'unité d'agitation (12) sont au moins partiellement
par moulage par injection ou par un procédé de fabrication additive.

8. Agitateur (10) selon au moins l'une des revendications précédentes, comprenant
- au moins un agitateur à disque disposé sur l'arbre d'agitateur (11) et espacé axialement de l'unité d'agitation (12), et/ou
- au moins un agitateur à pales inclinées disposé sur l'arbre d'agitateur (11) et espacé axialement de l'unité d'agitation (12), et/ou
- au moins une unité d'agitation supplémentaire disposée sur l'arbre d'agitation (11) et espacée axialement de l'unité d'agitation (12), comportant une section de montage de pale d'agitation supplémentaire et au moins une pale d'agitation supplémentaire montée sur ladite section de montage de pale d'agitation et pouvant se déplacer entre deux positions angulaires différentes dans lesquelles la au moins une pale d'agitation supplémentaire est alignée selon des angles d'attaque différents.

9. Unité d'agitation (12) destinée à l'équipement ultérieur d'un arbre d'agitation (11), comprenant
- une section de montage de pales (20) et
- au moins une pale d'agitation (13) disposée sur la section de montage de pale d'agitation (20) et mobile entre une première position angulaire et une deuxième position angulaire,
dans laquelle la au moins une pale d'agitation (13) est orientée selon un premier angle (24) dans la première position angulaire et selon un deuxième angle (25) dans la deuxième position angulaire, dans laquelle la au moins une pale d'agitation (13) comprend un élément rotatif (14) rigide en rotation qui est mobile entre une première position de rotation et une
deuxième position de rotation,
dans lequel la au moins une pale d'agitation (13) est alignée dans la première position angulaire selon le premier angle (24) et dans la deuxième position angulaire selon le deuxième angle (25),
dans lequel la section de montage de la pale d'agitation (20) comporte au moins une ouverture de montage (23) dans laquelle l'élément rotatif (14) est monté de manière rotative entre la première position de rotation et la deuxième position de rotation,
comprenant en outre
- une première butée qui empêche le mouvement de la au moins une pale d'agitation
(13) dans la première position angulaire dans une première direction de déplacement, et/ou
- une deuxième butée qui limite le mouvement de la au moins une pale d'agitation (13) dans la deuxième position angulaire dans une deuxième direction de déplacement, dans lequel, de préférence, la première butée et/ou la deuxième butée est prévue sur le
section de réception de la pale d'agitation, l'ouverture de réception (23) formant la première butée (21) et/ou la deuxième butée (22) ou étant délimitée par la première butée (21) et/ou la deuxième butée (22).

10. Bioréacteur (1) comprenant
- une plaque supérieure (2) et une cuve (3),
dans lequel la plaque supérieure (2) et la cuve (3) délimitent une chambre de réaction,
- un agitateur (10) disposé dans la chambre de réaction selon au moins l'une des revendications 1 à 8 précédentes,
dans lequel l'arbre d'agitateur (11) est monté de manière rotative dans un palier autour d'un axe de rotation,
- un arbre d'entraînement (12) disposé à l'extérieur de la chambre de réaction et couplé à l'arbre de l'agitateur (11),

11. Procédé de fabrication d'une unité d'agitation selon la revendication 9, et/ou d'un agitateur (40) selon au moins l'une des revendications 1 à 8, comprenant les étapes suivantes :
- fournir une section de montage de pale d'agitateur (41),
- fournir au moins une pale d'agitation (42),
- monter ladite au moins une pale d'agitation sur la section de montage de pale d'agitation (43) de telle sorte que ladite au moins une pale d'agitation soit mobile entre une première position angulaire et une deuxième position angulaire, de telle manière que ladite au moins une pale d'agitation, dans la première position angulaire, présente un premier angle d'attaque et, dans la deuxième position angulaire, présente un deuxième angle d'attaque.

12. Procédé de fabrication d'un bioréacteur (50), en particulier selon la revendication 10 précédente, comprenant les étapes consistant à :
- fournir un récipient (51),
- fournir une plaque supérieure (52),
- fournir un agitateur selon l'une des revendications 1 à 8 (53),
- monter l'agitateur sur la plaque supérieure (54),
- placer la plaque supérieure sur le récipient (55), et
- raccorder la plaque supérieure au récipient (56) de manière à former une chambre de réaction.

13. Procédé de fonctionnement d'un bioréacteur (60) selon la revendication 10 ci-dessus, comprenant un agitateur selon au moins l'une des revendications 1 à 8, et/ou une unité d'agitation selon la revendication 9, comprenant les étapes suivantes :
- ajuster un premier paramètre d'entraînement (61) selon les besoins pour l'arbre d'agitation dans un premier sens de rotation de sorte qu'au moins une pale d'agitation soit déplacée vers une première position angulaire dans laquelle ladite au moins une pale d'agitation est alignée selon un premier angle d'attaque, et/ou
- ajuster, en fonction de la demande, un deuxième paramètre d'entraînement (62) pour entraîner l'arbre d'agitation dans un deuxième sens de rotation, de telle sorte que la ou les
au moins une pale d'agitation soit déplacée vers une deuxième position angulaire dans laquelle ladite au moins une pale d'agitation est alignée selon un deuxième angle d'attaque,
dans lequel le premier paramètre d'entraînement et le deuxième paramètre d'entraînement sont réglables alternativement.
